(19) Europäisches Patentamt
European Patent Office
Office européen des brevets

(11) **EP 3 555 557 B1**

(12) **EUROPÄISCHE PATENTSCHRIFT**

(45) Veröffentlichungstag und Bekanntmachung des Hinweises auf die Patenterteilung:
**30.09.2020  Patentblatt 2020/40**

(21) Anmeldenummer: **17816639.3**

(22) Anmeldetag: **07.12.2017**

(51) Int Cl.:
*G01B 9/02* *(2006.01)*          *G02B 5/122* *(2006.01)*

(86) Internationale Anmeldenummer:
**PCT/EP2017/081832**

(87) Internationale Veröffentlichungsnummer:
**WO 2018/108697 (21.06.2018 Gazette 2018/25)**

(54) **ANORDNUNG UND VERFAHREN ZUR ROBUSTEN SINGLE-SHOT-INTERFEROMETRIE**

ARRANGEMENT AND METHOD FOR ROBUST SINGLE-SHOT INTERFEROMETRY

AGENCEMENT ET PROCÉDÉ POUR RÉALISER UNE INTERFÉROMÉTRIE ROBUSTE À INTERFÉROGRAMME UNIQUE

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **13.12.2016  DE 102016014802**

(43) Veröffentlichungstag der Anmeldung:
**23.10.2019  Patentblatt 2019/43**

(73) Patentinhaber: **Universität Stuttgart**
**70174 Stuttgart (DE)**

(72) Erfinder:
• **KÖRNER, Klaus**
  **10367 Berlin (DE)**
• **OSTEN, Wolfgang**
  **70569 Stuttgart (DE)**

(74) Vertreter: **Müller-Boré & Partner**
**Patentanwälte PartG mbB**
**Friedenheimer Brücke 21**
**80639 München (DE)**

(56) Entgegenhaltungen:
**US-A1- 2003 048 456      US-A1- 2011 235 056**
**US-A1- 2012 307 258      US-B1- 7 956 630**

EP 3 555 557 B1

**Beschreibung**

Die vorliegende Erfindung betrifft eine Anordnung und ein Verfahren zur Single-shot-Interferometrie zur Erfassung von Abstand, Profil, Form, Welligkeit, Rauheit oder der optischen Weglänge in oder an optisch rauen oder glatten Objekten und/oder zur optischen Kohärenz-Tomografie (OCT).

Eine Anordnung und ein Verfahren zur Single-shot-Interferometrie sind z.B. aus der Patentschrift EP 2 843 360 A1 bekannt. Bei den in EP 2 843 360 A1 beschriebenen interferometrischen Ansätzen trifft eine Wellenfront-Inversion auf, die für ein fehlerarmes Messen auf der Basis von zueinander geneigten interferierenden Wellenfronten oder Wellenfronten mit Krümmung und Lateral-Shear besonders hohe Anforderungen an die vorgeordnete strahlformende Optik stellt. Diese Anforderungen bestehen insbesondere hinsichtlich geringer Wellenfront-Aberrationen. Weitere interferometrische Ansätze sind z.B. aus den Patentschriften EP 2 526 373 B1 und DE 10 2010 056 122 B3 bekannt. Diese Ansätze basieren auf Endreflektor-Anordnungen im Referenzstrahlengang eines Interferometers mit drei Planspiegeln und mit einem gekreuzten Strahlengang. In den Patentschriften EP 2 526 373 B1 und DE 10 2010 056 122 B3 wird jedoch kein Hinweis darauf gegeben, wie ein großer Aperturwinkel für eine vergleichsweise hohe numerische Apertur N.A. zu erreichen wäre, beispielsweise für eine numerische Apertur N.A. = 0,4. Eine numerische Apertur N.A. in diesem Größenbereich ist jedoch für das Sicherstellen einer hohen lateralen Auflösung im Bereich von einem Mikrometer und auch noch etwas darunter für sichtbares Licht unverzichtbar.

Ein Ziel der Erfindung liegt in der Bereitstellung von verbesserten Anordnungen und Verfahren für die gewerbliche Nutzung der Single-Shot-Interferometrie mit einem räumlichen Interferogramm, insbesondere auf der Basis mechanisch robuster optischer Interferometer-Komponenten.

Insbesondere liegt der Erfindung die Aufgabe zugrunde, die numerische Apertur einer interferometrischen Messanordnung insbesondere für die Single-Shot-Interferometrie mit einem räumlichen Interferogramm im Vergleich zum Stand der Technik signifikant zu erhöhen, wobei die Robustheit der Anordnung gewährleistet wird. Dies soll z.B. mit Endreflektor-Baugruppen in einem Zweistrahl-Interferometer ermöglicht werden, die sowohl klassisch optisch durch Poliertechniken als auch mit drucktechnischen Verfahren oder bei Luft-Spiegel-Baugruppen mittels metallischer Komponenten und mittels Single-Point-Diamantbearbeitung gefertigt werden können.

Eine weitere Aufgabe ist es daher, technisch gut herstellbare optische Baugruppen bereitzustellen, die robust genug auch für den Einsatz in einer Fertigungsumgebung sind und/oder auch für das Inline-Messen in einer mechanischen Fertigungslinie eingesetzt werden können. Des Weiteren soll die Aufgabe gelöst werden, eine Verschiebung in z-Richtung des Single-Shot-Interferometers zu messen.

Diese Aufgabe(n) wird bzw. werden durch eine Anordnung zur robusten Zweistrahl-Interferometrie nach Anspruch 1 und ein Verfahren zur robusten Zweistrahl-Interferometrie nach Anspruch 11 oder 15 gelöst. Bevorzugte Ausführungsformen sind Gegenstand der Unteransprüche.

Insbesondere werden eine Anordnung und ein Verfahren zur Single-shot-Interferometrie vorgeschlagen, die zur Erfassung von Abstand, Profil, Form, Welligkeit, Rauheit oder der optischen Weglänge in oder an optisch rauen oder glatten Objekten und/oder auch zur optischen Kohärenz-Tomografie (OCT) eingesetzt werden können. Die Anordnung umfasst eine Lichtquelle, ein Interferometer, bei dem im Referenzstrahlengang ein End-Reflektor angeordnet ist, sowie einen Detektor zur Detektion eines Interferogramms. Im Referenzstrahlengang des Interferometers kann der End-Reflektor mit drei planen Reflexionsflächen als Prismenspiegel- oder als Luftspiegel-Baugruppe ausgebildet werden, um zwischen Referenz- und Objektbündel eine Lateral-Shear vom Betrag delta_q zur Gewinnung eines räumlichen Interferogramms zu erzeugen. Die Ausbildung dieser Baugruppe hinsichtlich der Winkel und der Anordnung der Reflexionsflächen ermöglicht einen großen Aperturwinkel für eine hohe numerische Apertur. Beim Verfahren kann im Referenzstrahlengang eine Reduzierung des Aperturwinkels des Referenz-Strahlenbündels mit bündelbegrenzenden Mitteln durchgeführt werden, um eine optimale Anpassung an den geometrisch gegebenen Aperturwinkel des End-Reflektors im Referenzstrahlengang zu erreichen, der kleiner als der Aperturwinkel im Objektstrahlengang ausgebildet ist. Der End-Reflektor im Referenzstrahlengang kann auch als Bestandteil eines zweiten Interferometers zur hochaufgelösten Messung der Verschiebung der Anordnung zur Single-shot- Interferometrie genutzt werden, wobei diese Verschiebung der Fokussierung dient.

Der End-Reflektor ist als eine Dreifach-Reflexions-Anordnung (z.B. eine Prismenanordnung) mit drei Reflexionsflächen ausgebildet. Die Dreifach-Reflexions-Anordnung kann einen M- oder W-Strahlengang, einen sich nicht kreuzenden Zick-Zack Strahlengang oder einen sich kreuzenden (Zick-Zack) Strahlengang aufweisen.

Insbesondere wird gemäß einer ersten Ausführung eine Anordnung zur robusten Zweistrahl-Interferometrie bereitgestellt, z.B. eine Anordnung zur Erfassung von Abstand, Tiefe, Profil, Form, Welligkeit und/oder Rauheit oder der optischen Weglänge in oder an rauen oder optisch glatten technischen oder biologischen Objekten, auch in Schichtenform, und/oder zur optischen Kohärenz-Tomografie (OCT).

Die Anordnung umfasst folgende Mittel:

eine Quelle kurzkohärenter elektromagnetischer Strahlung zur Beleuchtung des Objekts,

ein Interferometer, insbesondere auch in Form eines Interferenzmikroskops, mit einem Objektstrahlengang und mit mindestens einem Referenzstrahlengang und einer Messebene im Objektstrahlengang, in der sich zumindest näherungsweise die optisch anzumessenden Oberflächen- oder Volumenelemente des Objekts befinden; sowie mindestens einen gerasterten Detektor zur Detektion elektro-magnetischer Strahlung in Form mindestens eines räumlichen Interferogramms, wobei:

im Referenzstrahlengang des Interferometers mindestens ein End-Reflektor als Referenzreflektor angeordnet ist, wobei der End-Reflektor als eine Dreifach-Reflexions-Anordnung mit drei Reflexionsflächen ausgebildet.

Die drei Reflexionsflächen liegen jeweils zumindest näherungsweise senkrecht zu einer gemeinsamen Bezugsebene BE. Die drei Spurgeraden der Ebenen, welche durch die drei Reflexionsflächen dargestellt werden, bilden in der Bezugsebene BE ein Dreieck ABC mit einem stumpfen Winkel, damit bei diesem Interferometer zwischen Referenz- und Objektbündel eine Lateral-Shear vom Betrag delta_q besteht. Die erste Reflexionsfläche liegt auf einer Geraden, auf welcher die Punkte C und B liegen, die zweite Reflexionsfläche liegt auf einer (verlängerten) Geraden, auf welcher die Punkte A und C liegen, und die dritte Reflexionsfläche liegt auf einer (verlängerten) Geraden, auf welcher die Punkte A und B liegen. Die drei Reflexionsflächen können plane Flächen, z.B. Planspiegelflächen sein.

Ferner werden die folgenden Merkmale eingehalten:
Der Strahlengang der Dreifach-Reflexions-Anordnung ist gekreuzt.

Die zweite Reflexionsfläche, liegend auf einer verlängerten Geraden m, welche die Punkte A und C enthält, wird als zweite (in der Reflexionsreihenfolge) der drei Reflexionsflächen zur Reflexion eines fokussierten Bündels FB genutzt.

Die erste Reflexionsfläche, welche die Punkte C und B enthält, und die zweite Reflexionsfläche sind in einem spitzen Winkel relativ zueinander angeordnet. Beispielsweise kann an der zweiten Reflexionsfläche die Reflexionsfläche, welche die Punkte C und B enthält, mit spitzem Winkel anliegen. Somit ist der Winkel ACB ein stumpfer Winkel.

Die erste (plane) Reflexionsfläche, welche die Punkte C und B enthält, wird als erste oder dritte (in der Reflexionsreihenfolge) der drei planen Reflexionsflächen zur Reflexion des Bündels FB genutzt.

Ferner gibt es eine Normale N_m vom Punkt B auf die zweite Reflexionsfläche, durch welche die Gerade m verläuft.

Die erste Reflexionsfläche, welche die Punkte C und B enthält, und die dritte Reflexionsfläche, welche die Punkte A und D enthält, bilden einen stumpfen Winkel CBD. Somit ist der Winkel ABC ein spitzer Winkel.

Es besteht für die Dreifach-Reflexions-Anordnung mit dem gekreuztem Strahlengang ein Winkel gamma zwischen Randstrahl RAS des ein- oder des austretenden Bündels und der zweiten Reflexionsfläche und dabei gilt für den Betrag des Winkels gamma < 12° (Altgrad).

Weiterhin gilt für den Betrag des Winkels gamma bevorzugt < 5° (Altgrad). Des Weiteren gilt für den Winkel gamma bevorzugt zumindest näherungsweise 0° (Altgrad). Letzteres ergibt einen vergleichsweise großen Aperturwinkel des fokussierten Strahlenbündels, welches die Dreifach-Reflexions-Anordnung noch passieren kann.

Weiterhin kann der Bündelfokus BF bevorzugt zumindest näherungsweise auf der Normalen N_m und zumindest näherungsweise in der Nähe der zweiten Reflexionsfläche liegen.

Weiterhin kann die Dreifach-Reflexions-Anordnung als Luftspiegelgruppe oder als Prismenspiegelgruppe ausgebildet sein.

Für das Prisma kann dafür ein Glaswerkstoff eingesetzt werden, der auch bevorzugt höherbrechend sein kann, um einen möglichst hohen Aperturwinkel für das fokussierte Bündel in der Prismenspiegelgruppe verarbeiten zu können. Beispielsweise kann der Brechungsindex des Glaswerkstoffs um 1,85 sein. Dies ist besonders für den Einsatz in einem Mirau-Interferometer mit einem Objektiv mit einer numerischen Apertur von 0,55 von Interesse. Die Prismenspiegelgruppe kann als ein Mini- oder Mikro-Prisma, d.h. als eine refraktive Komponente mit drei Reflexionen ausgebildet werden. Ein derartiges Mini- oder Mikro-Prisma ist vergleichsweise einfach zu fertigen und stellt einen Monolithen dar.

Die Luftspiegelgruppe lässt sich in der Regel nicht als ein Monolith fertigen, sondern als eine zusammengefügte Baugruppe. Eine solche zusammengefügte Baugruppe baut - bei gleichen Parametern - in der Regel größer als ein monolitisches Mini- oder Mikro-Prisma bzw. größer als eine refraktive Komponente.

Die vom fokussierten Bündel FB genutzte Reflexionsfläche auf einer verlängerten Geraden k, welche die Punkte A und B enthält (dritte Reflexionsfläche), ist bevorzugt mehr als dreifach so lang ausgebildet wie die zweite Reflexionsfläche auf einer verlängerten Geraden m, welche die Punkte A und C enthält.

Weiterhin ist die Dreifach-Reflexions-Anordnung bevorzugt mit einem vorzeichenbehafteten Winkel tau kleiner als -1° ausgebildet, also -2° bis -20°. Der Winkel tau ist der Winkel zwischen dem Eingangshauptstrahl in den Endreflektor bzw. in die Dreifach-Reflexions-Anordnung und der zweiten Reflexionsfläche.

Es handelt sich in einer weiteren Ausführung um eine Anordnung zur robusten Zweistrahl-Interferometrie zur Erfassung von Abstand, Tiefe, Profil, Form, Welligkeit und/oder Rauheit oder der optischen Weglänge in oder an technischen oder biologischen Objekten, auch in Schichtenform, und/oder zur optischen Kohärenz-Tomografie (OCT). Die Anordnung umfasst folgende Mittel:

eine Quelle kurzkohärenter elektromagnetischer Strahlung zur Beleuchtung des Objekts,
ein Interferometer, insbesondere auch in Form eines Interferenzmikroskops, mit einem Objektstrahlengang (O), mindestens einem Referenzstrahlengang (R) und einer Messebene im Objektstrahlengang, in der sich zumindest näherungsweise die optisch anzumessenden Oberflächen- oder Volumenelemente des Objekts befinden, mindestens einen gerasterten Detektor zur Detektion elektro-magnetischer Strahlung in Form mindestens eines räumlichen Interferogramms,

wobei:

im Referenzstrahlengang (R) des Interferometers mindestens ein End-Reflektor als Referenzreflektor angeordnet ist, wobei der End-Reflektor als eine Dreifach-Reflexions-Anordnung mit drei Reflexionsflächen ausgebildet ist, und die drei Reflexionsflächen jeweils zumindest näherungsweise senkrecht zu einer gemeinsamen Bezugsebene BE liegen, und die drei Spurgeraden der Ebenen, welche durch die drei Reflexionsflächen (die z.B. plane Spiegelflächen sein können) dargestellt werden, in der Bezugsebene BE ein Dreieck ABC mit einem stumpfen Winkel bilden. Dabei liegt die erste Reflexionsfläche auf einer (verlängerten) Geraden, auf welcher die Punkte C und B liegen, die zweite Reflexionsfläche auf einer (verlängerten) Geraden m, auf welcher die Punkte A und C liegen und die dritte Reflexionsfläche auf einer (verlängerten) Geraden k, auf welcher die Punkte A und B liegen.

Im Folgenden werden die kennzeichnenden Merkmale für einen möglichst großen Aperturwinkel alpha eines fokussierten Bündels für alle Dreifach-Reflexions-Anordnungen mit Planspiegelflächen genannt: Die Dreifach-Reflexions-Anordnung ist als Prismenspiegelgruppe ausgebildet.

Die Dreifach-Reflexions-Anordnung ist mit einem gekreuzten Strahlengang ausgebildet.

Die Reflexionsfläche, liegend auf einer verlängerten Geraden m, welche die Punkte A und C enthält (die zweite Reflexionsfläche), wird als zweite der drei Reflexionsflächen (in der Reihenfolge der Reflexionen) zur Reflexion eines fokussierten Bündels FB genutzt.

Die erste Reflexionsfläche und die zweite Reflexionsfläche sind in einem spitzen Winkel relativ zueinander angeordnet. Beispielsweise kann an der zweiten Reflexionsfläche die erste Reflexionsfläche, welche die Punkte C und B enthält, mit spitzem Winkel anliegen. Somit ist der Winkel ACB ein stumpfer Winkel.

Die Reflexionsfläche, welche die Punkte C und B enthält (die erste Reflexionsfläche), wird als erste oder als dritte (in der Reihenfolge der Reflexionen) der drei Reflexionsflächen zur Reflexion genutzt.

Es gibt eine Normale N_m vom Punkt B, gelotet auf die zweite Reflexionsfläche, durch welche die Gerade m verläuft.

Die erste Reflexionsfläche, welche die Punkte C und B enthält, und die dritte Reflexionsfläche auf einer verlängerten Geraden k, welche die Punkte A und B enthält, bilden einen stumpfen Winkel. Somit ist der Winkel ABC ein spitzer Winkel.

Es besteht für die Dreifach-Reflexions-Anordnung mit dem gekreuzten Strahlengang ein Winkel gamma zwischen Randstrahl RAS des ein- oder des austretenden Bündels und der zweiten Reflexionsfläche und dabei gilt für den Betrag des Winkels gamma <12° (Altgrad).

Weiterhin liegt der Bündelfokus BF bevorzugt zumindest näherungsweise auf der Normalen N_m und zumindest näherungsweise in der Nähe der zweiten Reflexionsfläche.

Weiterhin ist die Lichtquelle bevorzugt als Frequenzkamm-Laser mit einer Mikro-Kavität ausgebildet.

Weiterhin gibt es im Objektmessfeld bevorzugt mindestens ein Tiefenmesssystem zum Erfassen des Messobjekts in einer vorgegebenen (z.B. gröberen) Skala, wobei das Tiefenmesssystem mit seinem Strahlengang zumindest näherungsweise koaxial zum interferometrischen Strahlengang angeordnet ist. Das Tiefenmesssystem ist bevorzugt chromatisch-konfokal ausgebildet.

Weiterhin kann dem Interferometer im Referenzarm ein Abschwächungsfilter mit einem Maximum der Transmission im Zentrum des Abschwächungsfilters zur Verringerung des Aperturwinkels des Referenzstrahlenbündels zugeordnet werden.

Das Abschwächungsfilter im Referenzarm dient dazu, den Durchmesser des Referenzstrahlenbündels oder den Aperturwinkel des Referenzstrahlenbündels so zu verkleinern, dass die vom Referenz-Endreflektor durch seine Geometrie und gegebenenfalls den Brechungsindex bei einer Prismenausführung der Drei-Reflexions-Baugruppe vorgegebene numerische Apertur nicht überschritten wird. Bevorzugt kann die numerische Apertur im Referenzarm des Interferometers dabei auf die Hälfte verringert werden. Das führt bei einer Interferometeranordnung mit einer vergleichsweise hohen numerischen Apertur im Objektarm jedoch zu keinem Nachteil, da aufgrund der geringen wellenoptischen Schärfentiefe und des Abgleichs der optischen Weglängen das räumliche Interferogramm etwa in der Mitte der zueinander geneigten Wellenfronten auftritt. Wegen der bevorzugt kurzkohärenten Lichtquelle liegen die auftretenden signifikanten Modulationen im räumlichen Interferogramm in der Anzahl unter 20.

Die Kippung der beiden interferierenden Wellenfronten ist dabei durch die Größe der Lateral-Shear delta_q bevorzugt so groß gemacht, dass die Ausdehnung des räumlichen Interferogramms bevorzugt weniger als ein Drittel der lateralen Ausdehnung der Objektwellenfront beträgt.

Weiterhin ist das Abschwächungsfilter bevorzugt mit radialsymmetrischer Gauß-Charakteristik ausgebildet. Das ist für die Ausbildung der Wellenfronten von Vorteil.

Weiterhin ist das Abschwächungsfilter bevorzugt mit einer eindimensionalen Charakteristik ausgebildet.

Auf die Reflexionsfläche einer Drei-Reflexions-Baugruppe bzw. Anordnung, welche die Punkte B und D enthält, besteht bevorzugt ein streifender Einfall zumindest für den Randstrahl eines Bündels mit einem Einfallswinkel größer als 75° (Altgrad). So kann für das Referenzstrahlenbündel ein vergleichsweise großer Aperturwinkel, beispielsweise von 17,5° (Altgrad) und somit eine vergleichsweise große numerische Apertur N.A., von 0,3 bei einer Luftanordnung erreicht werden.

Weiterhin können das Interferometer, der Detektor und optional andere optische Elemente innerhalb eines Sensorkopfes angeordnet sein. Dem Sensorkopf ist bevorzugt ein hochdynamisches Stell-System mit einem beigeordneten hochauflösenden Tiefenmesssystem zugeordnet, wobei das Stell-System in Mess-Echtzeit den Sensorkopf an jedem kooperativen Messpunkt stets im wellenoptischen Schärfentiefebereich hält. Das Stell-System ist bevorzugt ein Piezosteller-System, der das Steuersignal vorzugsweise aus dem WLI-Signal (WLI: Weißlicht Interferogramm) erhält. Vorzugsweise wird aber auch das Tiefenmesssignal eines in einer vorgegebenen (z.B. gröberen) Tiefenmess-Skala arbeitenden vorzugsweise chromatisch-konfokalen Sensors, der koaxial zum Messstrahlengang angeordnet ist, genutzt. Die Trennung der Signale erfolgt durch Farbteiler.

Weiterhin liegt der Winkel kappa in einem Michelson-Interferometer bevorzugt zwischen 96° (Altgrad) und 140° (Altgrad). Der Winkel besteht zwischen optischer Achse des Objektstrahlengangs im Interferometer und optischer Achse des Strahlengangs am Ausgang des Interferometers. Eine von 90° (Altgrad) abweichende Interferometer-Konfiguration schafft mehr Bauraum am Ausgang des Interferometers.

Weiterhin ist vorzugsweise zwecks Justierung ein lateral schiebbarer (z.B. schlanker) Glaskeil mit einer Mittendicke d_Pr im Objektarm und auf der optischen Achse des Mikroskop-Objektivs eines Zweistrahl-Interferometers angeordnet, mit welchem die Glasweglänge des Referenzarms des Zweistrahl-Interferometers zu null kompensiert wird. Die maximale Dicke d_Pr des Glaskeils kann im Bereich von 1mm bis 2 mm liegen. Bei einem stabilen Aufbau des Interferometers muss diese Justierung nur einmal erfolgen.

Vorzugsweise - bei Anordnung eines Drei-Reflexionsflächen-Prismas im Referenzarm - entspricht diese Mittendicke d_Pr des schiebbaren Glaskeils zumindest näherungsweise der Glasweglänge des Drei-Reflexionsflächen-Prismas. Durch eine Justierung mit Hilfe des Glaskeils kann der optische Gangunterschied (OPD) des Referenzarms und des Objektarms minimiert und z.B. auf kleiner als $2\mu$m reduziert werden. So besteht ein hinsichtlich des optischen Gangunterschieds sehr gut abgeglichenes balanciertes Interferometer, welches zumindest an achssenkrechten Spiegelflächen ein symmetrisches räumliches Weißlicht-Interferogramm (rWLI) liefern kann.

Gemäß einem weiteren Aspekt der Erfindung wird ein Verfahren zur robusten Zweistrahl-Interferometrie zur Erfassung von Abstand, Tiefe, Profil, Form, Welligkeit und/oder Rauheit oder der optischen Weglänge in oder an technischen oder biologischen Objekten, auch in Schichtform, oder auch zur optischen Kohärenz-Tomografie (OCT) vorgeschlagen.

Das Verfahren umfasst das Bereitstellen einer Anordnung zur robusten Zweistrahl-Interferometrie, welche eine der zuvor beschriebenen Anordnungen ist.

Ferner wird im Referenzstrahlengang eine Reduzierung des Aperturwinkels des Referenz-Strahlenbündels mit bündelbegrenzenden Mitteln durchgeführt. Die Reduzierung des Aperturwinkels des Referenzstrahlenbündels wird vorzugsweise durch eine Strahlabschwächung mit einem radialsymmetrischen Filter oder einem eindimensionalen Filter erreicht.

Dies ist von Vorteil, denn so kann bei diesem Interferometer ein Referenz-Endreflektor verwendet werden, der eine deutlich geringere numerische Apertur verarbeiten kann als im Objektstrahlengang gegeben ist. Die hohe numerische Apertur im Objektstrahlengang wird für die interferometrische Messanordnung nur für das Objekt-Strahlenbündel zur Bildung eines feinen beugungsbegrenzten Abtastflecks auf dem Objekt benötigt. Dies ist von größter Wichtigkeit bei feinbearbeiteten Oberflächen im Maschinenbau, die keine Spiegel darstellen.

Die Reduzierung des Aperturwinkels des Referenz-Strahlenbündels mit bündelbegrenzenden Mitteln wird dabei bevorzugt zumindest näherungsweise in der Fourier-Ebene eines Objektivs im Referenzstrahlengang eines Linnik-Interferometers durchgeführt. So wird die Abschattung der Bündel minimiert, da in der Fourier-Ebene Strahlenbündel, die im Objektraum eine Lateral-Shear aufweisen, sich überdecken.

Um das Balance-Signal bzw. Regelsignal für die Fokus-Regelung des (Weißlicht-) Interferometers zu bilden, können die Beträge I_links und I_rechts der Intensitäten benachbarter Fotoelemente eines Fotodioden-Detektors oder Pixel eines gerasterten Detektors, die sich vom räumlichen Weißlicht-Interferogramm links und rechts von einem Referenzpunkt RP befinden, mittels elektronischem Rechenwerk bestimmt und aufsummiert werden. Der Referenzpunkt RP liegt in der Regel auf der optischen Achse. Die Summe S_links der Beträge I_links und die Summe S_rechts der Beträge I_rechts werden voneinander subtrahiert und daraus wird das vorzeichenbehaftete Regelsignal (Balance-Signal) abgeleitet. Vorzugsweise wird auf null geregelt. Das elektronische Rechenwerk kann als ein digitaler Signalprozessor ausgebildet sein.

Das Interferometer, der Detektor und optional weitere optische Elemente können innerhalb eines Sensorkopfes angeordnet sein. Dem Sensorkopf sind vorzugsweise elektromechanische hochdynamische Mittel zur Tiefen-Nachführung des Sensorkopfes zugeordnet, die in Mess-Echtzeit den Sensorkopf an jedem kooperativen Messpunkt stets im wellenoptischen Schärfentiefebereich halten. Das Messergebnis wird stets aus der vorzeichenbehafteten Addition des Tiefenmesswertes des Nachführ-Tiefenmesssystems und des Tiefenmesswertes z_rWLI gebildet. Der Tiefenmesswert z_rWLI wird mittels räumlichen Weißlicht-Interferogramms bzw. aus dem räumlichen Weißlicht-Interferogramm-Signal oder rWLI-Signal ermittelt.

Bei einem weiteren Verfahren zur robusten Zweistrahl-Interferometrie zur Erfassung von Abstand, Tiefe, Profil, Form, Welligkeit und/oder Rauheit oder der optischen Weglänge in oder an technischen oder biologischen Objekten, auch in Schichtform, oder auch zur optischen Kohärenz-Tomografie (OCT) mit Bildung eines räumlichen Weißlicht-Interferogramms, umfassend:

Bereitstellen einer Anordnung zur robusten Zweistrahl-Interferometrie, welche eine der zuvor beschriebenen Anordnungen ist.

Ferner wird eine zweite optische Antastung des End-Reflektors mit den drei Reflexionsflächen mittels eines zweiten separaten Interferometers mit einer Laserlichtquelle durchgeführt, um eine Verschiebung in z-Richtung des zum End-Reflektor gehörenden ersten Interferometers messen zu können. Das Interferometer ist mit dem End-Reflektor starr verbunden und wird als Single-Shot-Interferometer betrieben. Das Single-Shot-Interferometer mit der Quelle kurzkohärenter elektromagnetischer Strahlung ist bevorzugt als Mirau-Interferometer ausgebildet. Im Referenzarm des zweiten separaten Interferometers ist bevorzugt ein Tripelreflektor in Form einer Raumecke angeordnet. Der Referenzarm des zweiten separaten Interferometers ist bevorzugt mit einem Strahlteiler ausgebildet, der im ersten Interferometer nur als Ein- und Auskoppelstrahlteiler genutzt wird. Die interferierenden Bündel des zweiten Interferometers verlassen dabei den Strahlteiler wieder auf dessen Eingangsseite. Mit diesem Ansatz kann die Verschiebung z_1 des ersten Interferometers - als kompakte Anordnung - in z-Richtung gemessen werden. So kann aus dem Wert z_WLI, welcher vom ersten Interferometer bestimmt wird, und dem z_1 Wert eine Position für einen Objektpunkt in Bezug auf eine Komponente des zweiten separaten Interferometers errechnet werden, beispielsweise in Bezug auf einen im optischen System angeordneten Strahlteiler des zweiten separaten Interferometers, welcher im ersten Interferometer als Ein- und Auskoppelstrahlteiler genutzt wird. Der z_1 Wert ergibt sich aus der Veränderung des optischen Gangunterschiedes im zweiten Interferometer, welche zeitaufgelöst gemessen wird und für den Fachmann in bekannter Weise in ein Wegsignal umgerechnet werden kann. Beim Starten des Messvorganges mit dem ersten Interferometer kann der z_1 Wert auf null gesetzt werden. Anschließend wird kontinuierlich die Veränderung des optischen Gangunterschieds bestimmt. Es kann beim Starten des Messvorganges mit dem ersten Interferometer aber auch ein beliebiger Positionswert in Millimetern dem z_1 Wert zugewiesen werden. Eine weitere Möglichkeit besteht darin, dem zweiten Interferometer auch noch eine Weißlicht-Quelle zuzuordnen und aus dem Weißlicht-Interferogramm am optischen Gangunterschied null ein Referenz-Signal zu generieren.

## Weitere Merkmale und/oder Vorteile der Erfindung

Vorzugsweise werden weitere Single-shot-Messpunkte eingesetzt, bevorzugt nach dem chromatisch-konfokalen Ansatz, um den interferometrischen Messpunkt oder die interferometrischen Messpunkte mittels schnellem Regel-System für den Messabstand in einem vergleichsweise kleinen Messbereich zu halten, da dieser Messabstand bei einer höheren numerischen Apertur nur wenige Mikrometer Tiefenbereich aufweist.

Bevorzugt kann auch ein ganzes Feld von Single-Shot-Messpunkten in Linien- oder Matrixanordnung, bevorzugt nach dem chromatisch-konfokalen Ansatz, also mit chromatisch-konfokalen Sensoren zusätzlich abgetastet werden. Dabei ist die Tiefenauflösung eines chromatisch-konfokalen Sensors bevorzugt besser als ein Fünftel des wellenoptischen Schärfentiefebereichs und der Messbereich beträgt bevorzugt mindestens das Fünffache des wellenoptischen Schärfentiefebereichs des Strahlenganges im Objektarm des Interferometers.

Die Anordnungsmerkmale der Single-Shot-Messpunkte in Matrixanordnung, bevorzugt nach dem chromatisch-konfokalen Ansatz, basieren bevorzugt auf Merkmalen des Schutzrechtes DE 10 2006 007172 B4.

Bevorzugt ist die Tiefenauflösung eines chromatisch-konfokalen Sensors besser als ein Zehntel des wellenoptischen Schärfentiefebereichs und der Messbereich beträgt mindestens das 100-fache des wellenoptischen Schärfentiefebereichs.

Eine linienhafte Single-Shot-Messung wird mit den vorgeschlagenen Anordnungen bei entsprechender Ausbildung des Sensors mit Zylinder-Optik bevorzugt mit einer linienhaften Licht-Quelle sowie einer Zylinder-Optik und einer schneller Flächenkamera durchgeführt.

Ein chromatisch-konfokaler Sensor ist bevorzugt koaxial zum interferometrischen Sensor-Strahlengang im Objektraum angeordnet.

Ein chromatisch-konfokaler Sensor ist bevorzugt als Vorlauf-Sensor im interferometrischen Sensor-Strahlengang im Objektraum angeordnet. Damit bekommt das Regelungs-System auf einer Drei-Koordinaten-Messmaschine vorab schon Informationen über die bei der anstehenden Messfahrt über das Messobjekt zu erwartenden Messabstände und der interferometrische Sensor-Kopf wird im Abstand stets rechtzeitig, also in Prozess-Echtzeit, darauf eingestellt.

Des Weiteren ist bevorzugt eine Zeile oder Matrix von chromatisch-konfokalen Punktsensoren, die im Vergleich zum interferometrischen Messen in einer gröberen Skala arbeiten, angeordnet. Die damit gewonnenen Informationen dienen u.a. der Abstandsregelung in Prozess-Echtzeit und der "Erkundung" des Messumfeldes in Prozess-Echtzeit.

Bevorzugt wird zusätzlich eine CMOS-Flächenkamera zur Beobachtung des Messfeldes eingesetzt.

Wie auch in EP 2 626 373 B1 bereits beschrieben, kann dieser Ansatz mit einem Frequenzkamm-Laser mit einer miniaturisierten Resonator-Kavität betrieben werden. Das hat den Vorteil, dass auch ein - hinsichtlich der optischen Weglänge - nicht ausbalanciertes Interferometer verwendet werden kann, da die benötigte Kompensation der optischen Weglänge - vereinfacht gesprochen - in den Frequenzkamm-Laser gelegt wird.

Die Erfindung wird nachfolgend beispielhaft anhand der Figuren 1 bis 14 sowie der 6 aufgeführten Ausführungsbeispiele ohne Figur beschrieben. Es zeigen:

**Fig.1** eine schematische Darstellung einer beispielhaften interferometrischen Anordnung zur robusten Zweistrahl-Interferometrie mit einem Michelson Interferometer und einer als ein Drei-Reflexionsflächen-Prisma ausgebildeten Dreifach-Reflexions-Anordnung;

**Fig. 2** eine Ansicht des Drei-Reflexionsflächen-Prismas in der Fig. 1;

**Fig. 2a** die Einbaulage des Drei-Reflexionsflächen-Prismas in der Fig. 1;

**Fig. 3** ein weiteres beispielhaftes Drei-Reflexionsflächen-Prisma mit einer Strahlenbündelkreuzung;

**Fig. 4** eine schematische Darstellung einer beispielhaften interferometrischen Anordnung zur robusten Zweistrahl-Interferometrie mit einem Mirau-Interferometer und einer als ein Drei-Reflexionsflächen-Prisma ausgebildeten Dreifach-Reflexions-Anordnung;

**Fig. 5** eine schematische Darstellung einer beispielhaften interferometrischen Anordnung zur robusten Zweistrahl-Interferometrie mit einem Linnik-Interferometer, einer als ein Drei-Reflexionsflächen-Prisma ausgebildeten Dreifach-Reflexions-Anordnung und einem chromatisch-konfokalen Mess-System;

**Fig. 6** eine beispielhafte Dreifach-Reflexions-Anordnung in Luftausführung;

**Fig. 7** eine schematische Darstellung einer weiteren beispielhaften interferometrischen Anordnung zur robusten Zweistrahl-Interferometrie mit einem Michelson-Interferometer;

**Fig. 8** eine schematische Darstellung einer weiteren beispielhaften interferometrischen Anordnung zur robusten Zweistrahl-Interferometrie mit einem Michelson-Interferometer;

**Fig. 9** ein beispielhaftes, nicht beanspruchtes Drei-Reflexionsflächen-Prisma mit einem W-Strahlengang;

**Fig. 10** zeigt eine schematische Darstellung einer beispielhaften, nicht beanspruchten interferometrischen Anordnung zur robusten Zweistrahl-Interferometrie mit einem Linnik-Interferometer und mit dem in Fig. 9 gezeigten Drei-Reflexionsflächen-Prisma;

**Fig. 11** zeigt beispielhafte räumliche-Interferrogramme;

**Fig. 12** eine schematische Darstellung einer beispielhaften, nicht beanspruchten interferometrischen Anordnung mit einem Mirau-Interferometer und mit dem in Fig. 9 gezeigten Drei-Reflexionsflächen-Prisma;

**Fig. 13** ein beispielhaftes, nicht beanspruchtes Laserinterferometer zur optischen Antastung bzw. Abtastung eines Drei-Reflexionsflächen-Prismas;

**Fig. 14** eine beispielhafte Messung von Oberflächenprofilen und -Welligkeit, deren Profiltiefe die der wellenoptischen Schärfentiefe der interferometrischen Sensor-Anordnung übersteigt. In den Figuren werden gleiche Bezugszeichen für die gleichen oder ähnlichen Elemente verwendet. Ferner wird der Begriff Licht stets als Synonym für elektromagnetische Strahlung vom Terahertz-, über das Infrarot- bis zum tiefen UV-Spektrum verwendet.

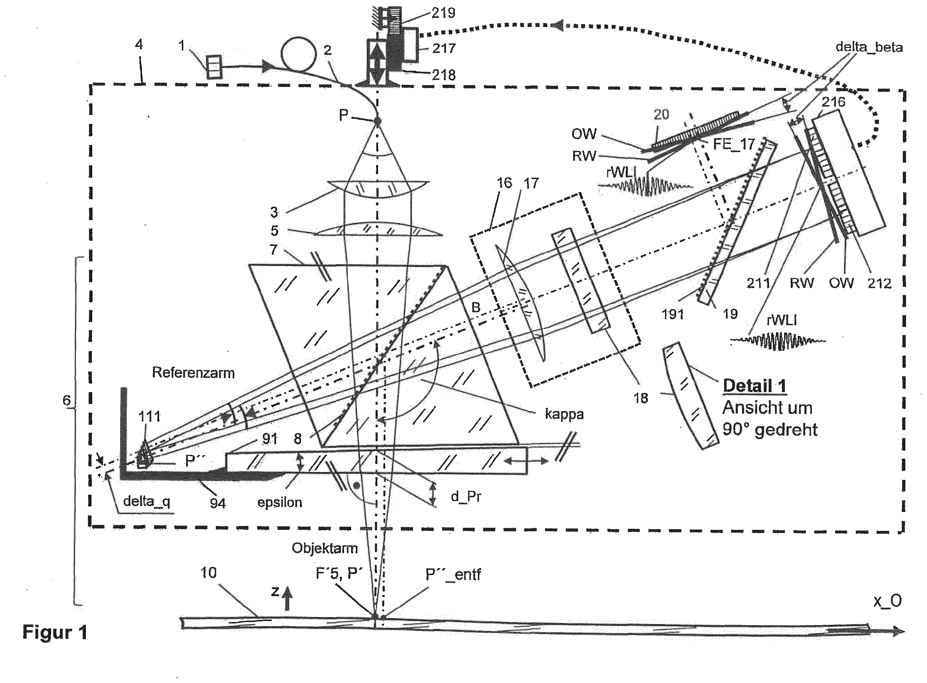

Die **Figur 1** stellt eine beispielhafte interferometrische Anordnung für das Single-Shot-Messen dar. Diese Anordnung ist insbesondere für Welligkeitsmessung an Dünnglas oder Messung der Ebenheitsabweichung an polierten Silizium-Wafern geeignet, da diese jeweils eine optisch glatte Oberfläche aufweisen und somit eine gerichtete Reflexion ohne Speckle-Effekte besteht. Das von der fasergekoppelten Punktlichtquelle 1 ausgehende Nah-infrarot-Licht gelangt über die Single-Mode-Faser 2 in den Kollimator 3, der bereits Bestandteil des Sensorkopfes 4 ist. Der Sensorkopf 4 umfasst ferner ein Michelson-Interferometer 6, eine Ausgangs-Abbildungsstufe 16, einen Detektor und gegebenenfalls weitere optische Elemente. Der Sensorkopf 4 ist in z-Richtung beweglich und präzisionsgeführt zur hochdynamischen Nachfokussierung ausgebildet. Die Punktlichtquelle 1 kann z.B. drei Superlumineszenz-Dioden umfassen, um die interferometrische Anordnung im spektralen Bereich von 750 nm bis 950 nm zu betreiben. Die Punktlichtquelle 1 kann auch eine andere Punktlichtquelle sein, z.B. ein Frequenzkamm-Laser mit einer Mikro-Kavität.

Die Abbildungsstufe für die Punktlichtquelle 1 stellt ein chromatisch voll auskorrigiertes optisches System vom Ende der Faser 2 bis zum Messobjekt 10 dar, das hier Dünnglas von 30 μm Dicke umfasst. Dabei sind die Glasweglängen und die Glasdispersion von der 112°-Strahlteileranordnung 7, dem Glaskeil 91 und dem Drei-Reflexionsflächen-Prisma 111 mittels des schiebbaren und dann fixierbaren Glaskeils 91 exakt kompensiert. Dazu ist der Keilwinkel epsilon des Glaskeils 91 in die 112°-Strahlteileranordnung 7 zumindest näherungsweise eingearbeitet, so dass im Abbildungsstrahlengang effektiv eine Planparallelplatte besteht und somit in der Abbildungsstufe keine spektrale Aufspaltung besteht.

Dieser Glaskeil 91 ist senkrecht zur optischen Achse vor dem Mikroskop-Objektiv ausgerichtet und hat auch eine Schutzfunktion. In Figur 1 beträgt die Dicke des Glaskeils 91 in der Mitte etwa 1,6 mm, in den Randbereichen etwa 1,3 mm und 1,9 mm, so dass eine sehr gute Balance der optischen Weglängen im Interferometer 6 eingestellt werden kann. So weist das Weißlicht-Interferogramm am Schwerpunkt der Einhüllenden zumindest näherungsweise die Phase null auf. Der einjustierte Glaskeil 91 und das Drei-Reflexionsflächen-Prisma 111 sind aus dem gleichen niedrig-dispersiven Glaswerkstoff hergestellt. Nach Justierung weisen die beiden Interferometerarme die gleichen Glasweglängen für den jeweiligen Hauptstrahl auf, so dass ein symmetrisches Weißlicht-Interferogramm entsteht. Das am Dünnglas 10 reflektierte Licht gelangt über den Glaskeil 91 zurück in die 112°-Strahlteileranordnung 7.

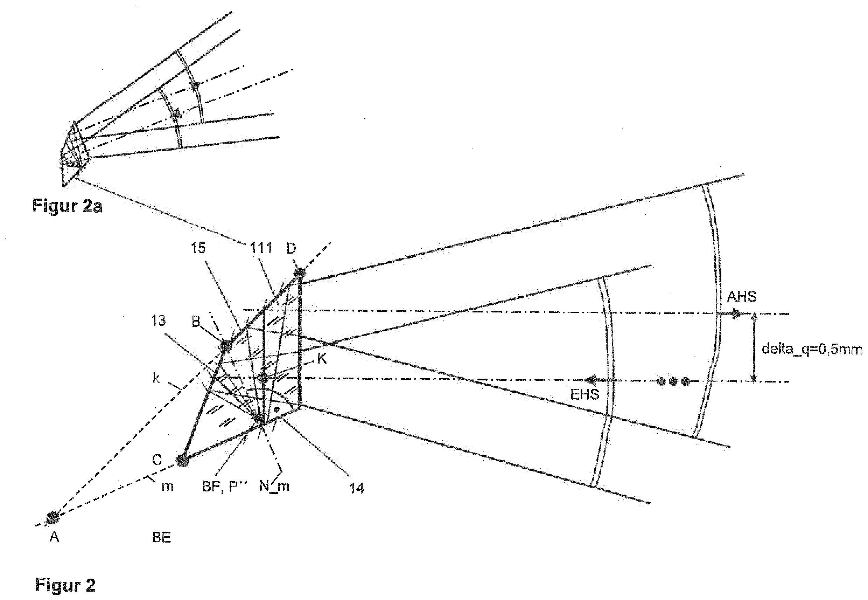

Im Referenzarm des Michelson-Interferometers 6 ist als Endreflektor ein Drei-Reflexionsflächen-Prisma 111 mit gekreuztem Strahlengang angeordnet. **Figuren 2** und **2a** zeigen schematisch den Aufbau des Drei-Reflexionsflächen-Prismas 111 (Figur 2) und deren Einbaulage (Figur 2a) im Interferometer.

Das Drei-Reflexionsflächen-Prisma 111 weist eine (Strahl-)Eingangsfläche 12 und drei Reflexionsflächen 13, 14 und 15 auf. Die drei Reflexionsflächen 13, 14 und 15 liegen jeweils zumindest näherungsweise senkrecht zu einer gemeinsamen Bezugsebene BE. Die Reflexionsflächen 13, 14 und 15 sind als planare Spiegel ausgebildet. Die drei Spurgeraden der Ebenen, welche durch die drei Reflexionsflächen dargestellt werden, bilden in der Bezugsebene BE ein Dreieck ABC mit einem stumpfen Winkel.

Die Reflexionsfläche 13 beinhaltet die Punkte B und C und wird als erste oder dritte Fläche (in der Reihenfolge der Reflexionen) zur Reflexion des Bündels genutzt. Die Reflexionsfläche 14 liegt auf einer verlängerten Geraden m, welche die Punkte A und C enthält, und wird als zweite Fläche (in der Reihenfolge der Reflexionen) zur Reflexion des Bündels benutzt. Der Winkel zwischen den Reflexionsflächen 13 und 14 ist ein spitzer Winkel. Die Reflexionsfläche 15 liegt auf einer verlängerten Geraden k, welche die Punkte A und B enthält. Die Reflexionsfläche 14 und die Reflexionsfläche 15 bilden einen stumpfen Winkel CBD.

Ferner verläuft eine Normale N_m auf die zweite Reflexionsfläche durch den Punkt B. Der Bündelfokus BF liegt zumindest näherungsweise auf der Normalen N_m und zumindest näherungsweise in der Nähe der Reflexionsfläche 14.

Das Drei-Reflexionsflächen-Prisma 111 ist für eine maximale numerische Apertur N.A. = 0,2 ausgebildet, die aber hier in Figur 1 nicht voll ausgenutzt wird. Mittels geometrischer Ausbildung des Drei-Reflexionsflächen-Prismas 111 ist ein Querversatz des Bündels delta_q = 500 μm gegeben. Das Mikroskop-Objektiv 5 besitzt eine Brennweite f'5= 40mm und eine numerische Apertur N.A. = 0,09, die voll ausgenutzt wird, so dass die numerische Apertur für das fokussierte Bündel hierbei N.A. = 0,09 beträgt. Damit ist der Pupillendurchmesser mit d_P mit 7,2 mm bestimmt. In der Pupillenebene ergibt sich somit ein maximaler optischer Gangunterschied OPD_max von delta_q * (d_P / f'5). Im vorliegenden Beispiel ist OPD_max = delta q * (d_P / f'5) = 500 μm * (7,2 mm / 40 mm) = 90 μm. Für eine Schwerpunkt-Wellenlänge von 850 nm ergeben sich somit etwa 106 Interferenzstreifen, die mit einer Zeilenkamera 20 mit 1024 Pixeln und 7 mm Länge gut detektiert werden können, wobei durch die Form der Einhüllenden des WLI-Wavelet (WLI: Weißlicht-Interferogramm), gegeben durch das Lichtspektrum der interferierenden Wellenfronten, nur jeweils deutlich weniger als 100 Perioden im WLI-Wavelet detektiert werden können. Die 112°-Strahlteileranordnung 7 gemäß Figur 1 gestattet auch die Nutzung von Mikroskop-Objektiven mit deutlich geringerer Brennweite als 40 mm, ohne dass sich Probleme mit dem freien Raum für die Komponenten des Detektions-Strahlenganges am Ausgang des Interferometers 6 ergeben.

Bei einer 112°-Strahlteileranordnung 7 mit 12 mm Kantenlänge und einer Dicke d_Pr des Glaskeils 91 von 1,6 mm, was in der Summe einer Deckglas-Korrektur von 13,6 mm entspricht, ergibt sich für das Mikroskop-Objektiven 5 mit einer Brennweite von 40 mm ein freier Arbeitsabstand von mindestens 15 mm, auch wenn sich die bildseitige Hauptebene im Mikroskop-Objektivs 5 etwas hinter der Frontlinse befindet. Am Ausgang des Interferometers 6 ist eine Ausgangs-Abbildungsstufe 16 angeordnet. Diese stellt ein chromatisch gut korrigiertes optisches Gesamtsystem vom Ausgang des Interferometers 6 bis zum Detektor 20 dar. Die darin enthaltene Zylinder-Optik 18 (siehe auch **Detail 1**) dient der linienförmigen Verdichtung des Strahlenbündels auf die schnelle Zeilen-Kamera 20, die das Interferenzlicht mittels einer Auskoppelstrahlteiler-Platte 19 erhält. Das transmittierte Interferenzlicht gelangt auf die schnellen Fotodioden-Detektoren 211 und 212. Der auf der linken Seite des Feldes angeordnete schnelle Fotodioden-Detektor 211 und der auf der rechten Seite des Feldes angeordnete schnelle Fotodioden-Detektor 212 dienen zur Erfassung der Fokuslage des Sensorkopfs 4 mit dem Interferometer 6. Mittels der Fotodioden-Detektoren 211 und 212, deren jeweilige Breite jeweils etwa einem Viertel der Periode im WLI-Wavelet entspricht, kann in bekannter Weise eine lokale Amplitude A des Weißlicht-Interferogramms zumindest näherungsweise nach folgender Formel aus 5 Fotodioden-Elementen bestimmt werden, welche die Signalwerte I1, I2, I3, I4 und I5 liefern.

$$A = \sqrt{\left(I_1 - 2I_3 + I_5\right)^2 + \left(2I_2 - 2I_4\right)^2} \qquad \text{Gleichung (1)}$$

Die Bestimmung der lokalen Amplitudenwerte A des Weißlicht-Interferogramms erfolgt auf jeder der beiden Seite des Feldes mehrfach und so wird für die linke und die rechte Seite mittels eines digitalen Signal-Prozessors 216 das Differenzsignal der Summen der ermittelten lokalen Amplitudenwerte gebildet, wobei für die Amplitudenwerte stets ein positiver Wert steht. Je nachdem, welche der beiden Seiten im Summensignal überwiegt, also eine positive oder negative Differenz besteht, wird in der Tiefe mittels eines hochdynamischen Piezosteller-Systems 218 der Sensorkopf mit Interferometer 6 zur Dünnglasoberfläche 10 nachgeregelt. Dieses Balance-Verfahren stellt für den Fachmann ein wohlbekanntes Verfahren für Regelungssysteme dar.

Es sind auch andere Gleichungen zur Bestimmung oder Abschätzung der lokalen Signalamplituden A eines Weißlicht-Interferogramms bekannt und ebenfalls anwendbar, welche beispielsweise nur auf der Differenz direkt benachbarter Intensitätswerte im räumlichen Interferogramm beruhen. Die Berechnung der Signalamplituden A kann mittels des digitalen Signal-Prozessors 216, welcher dem Fotodioden-Detektor zugeordnet ist und der parallel auf die Signale der Sensorelemente der Fotodioden zugreift, extrem schnell ausgeführt werden. Durch die Auswertung der Amplituden mehrerer Oszillationen unter der Einhüllenden des räumlichen Interferogramms kann vermieden werden, dass bei einer von der Gauß-Verteilungskurve abweichenden Form der Einhüllenden ein fehlerhaftes Signal entsteht, beispielsweise bei einer Modulation der Einhüllenden.

Die vergleichsweise große spektrale Bandbreite der Lichtquelle 1 von etwa 200 nm bewirkt eine vergleichsweise geringe Kohärenzlänge. Die Anordnung nach Figur 1 ist somit im Besonderen für Dünnglas-Messungen geeignet. Darüber hinaus ist die Anordnung nach Figur 1 auch für die Messung der Form polierter Asphären, einschließlich von Gleitsichtbrillen-Gläsern, auch aus Kunststoff verwendbar. Beim Messen von Gleitsichtbrillen-Gläsern kann die Anordnung nach Figur 1 Bestandteil einer Mehr-Koordinaten-Ultrapräzisions-Messmaschine sein. Diese ist u.a. mit einer ultrapräzisen Kipp-Vorrichtung, auch mit einem Kippwinkel-Sensor, aufgebaut, so dass stets ein zumindest näherungsweise senkrechtes Anmessen der Objektoberfläche durchgeführt werden kann.

Die Anordnung nach Figur 1 gestattet auch die Schichtdickenmessung an Dünnglas 10 mit der Dicke von beispielsweise 30 µm. In diesem Fall sind zwei deutlich separierte räumliche Weißlicht-Interferogramme auszuwerten. Somit können sowohl die Welligkeit, als auch die Dicke des Dünnglases 10 mit einer Tiefenauflösung im einstelligen Nanometerbereich gleichzeitig gemessen werden.

In einem ersten Ausführungsbeispiel (1) ohne Figur kann die Bestimmung einer lokalen Amplitude A des WLI-Wavelets sowohl auf der rechten Seite als auch auf der linken Seite der optischen Achse im Feld jeweils mit einer Vierfach-Fotodiode erfolgen. Dabei beträgt der Pitch der Vierfach-Fotodioden jeweils ein Viertel der bekannten mittleren WLI-Wavelet-Periodenlänge. Die Auswertung kann mittels speziellen Prozessors erfolgen. Dies setzt jedoch eine Einhüllende mit symmetrischer Form, die frei von lokalen Modulationen ist, voraus. Die Vierfach-Fotodioden bekommen das WLI-Licht mittels einer Auskopplung durch einen Strahlteiler. So erfolgt ein extrem schneller Vergleich einer linken und rechten Amplitude des räumlichen Interferogramms an einem festen Ort, um ein Defokus-Signal für die Tiefenregelung zu generieren.

Die Prismenanordnung 111 in Figur 2 mit drei Reflexionen und einem Kreuzungspunkt K findet z.B. Anwendung, wenn ein geringer Querversatz delta_q erreicht werden soll. Das kann für ein Michelson-, ein Linnik-oder auch ein Mirau-Interferometer von Vorteil sein, insbesondere dann, wenn die Brennweite der Mikroskop-Objektive gering ist. Diese Konfiguration weist eine kleine optische Weglänge im Verhältnis zum Querversatz delta_q auf. Im Glas der Prismenanordnung 111 wird ein halber Öffnungswinkel von 9° erreicht. Bevorzugt wird hierbei linear polarisiertes Licht verwendet.

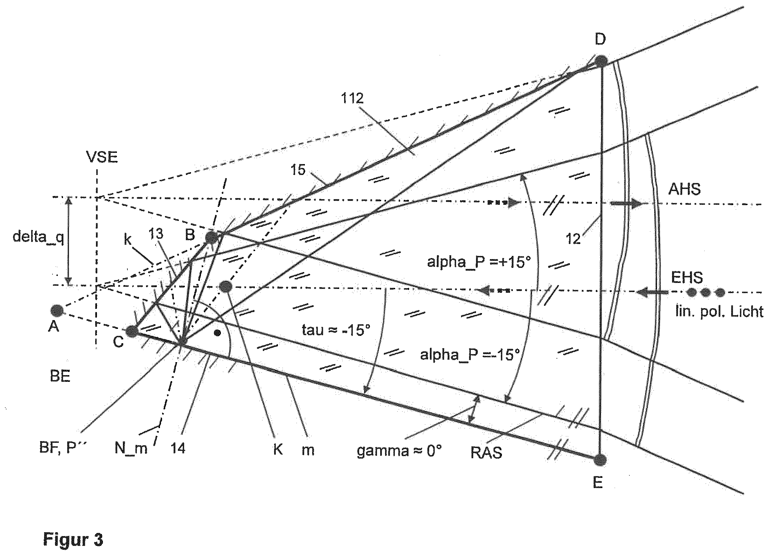

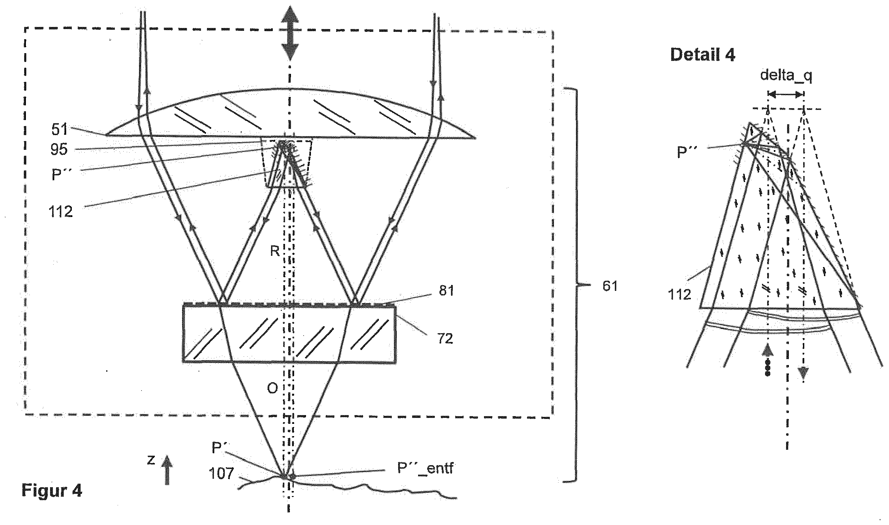

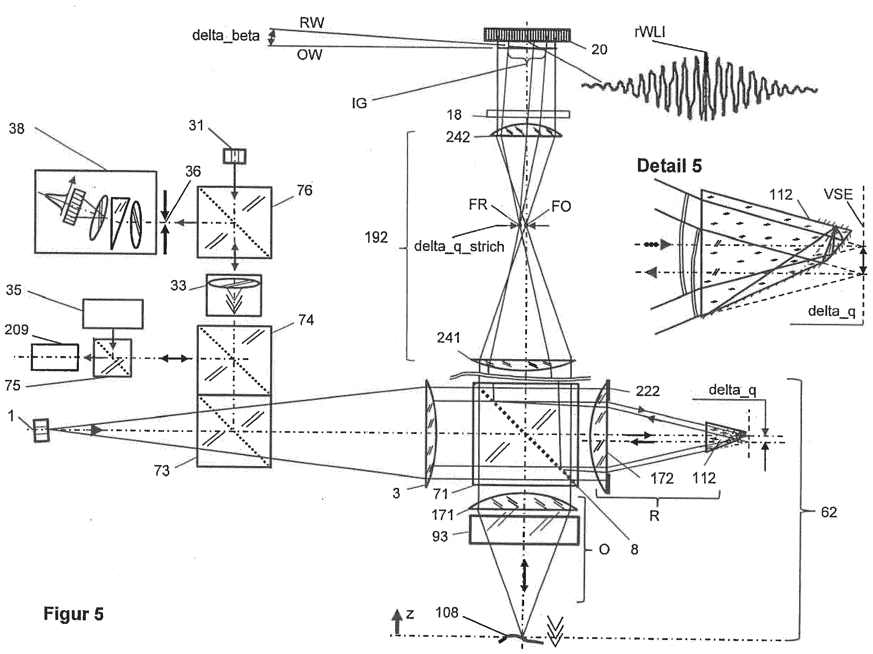

Die **Figur 3** stellt ebenfalls eine beispielhafte Prismenanordnung mit drei Reflexionen, also ein Drei-Reflexionsflächen-Prisma 112, mit einer Strahlenbündelkreuzung und einem Kreuzungspunkt K des Eingangsstrahlenbündes und des Ausgangsstrahlenbündes sowie der virtuellen Spiegelebene VSE dar. Diese spezielle Prismenanordnung findet Anwendung, wenn eine vergleichsweise hohe numerische Apertur N.A. für das fokussierte Bündel erreicht werden soll. Das Drei-Reflexionsflächen-Prisma 112 wird in Anordnungen gemäß der Figuren 4 und 5 eingesetzt. Es geht hier in der Figur 3 um die Maximierung der N.A. Für 15,35° wird eine N.A. von 0,4 für die Glassorte BK7 erreicht. Höherbrechende Gläser erlauben eine noch höhere N.A. von Werten bis zu 0,5. Der Winkel gamma beträgt hier etwa 0° (Altgrad). Dies stellt eine Voraussetzung für das Erreichen einer vergleichsweise hohen N.A. dar. Die hier dargestellte Variante mit einem halben Aperturwinkel von 15° (Altgrad) führt jedoch zu einem vergleichsweise großen Bauvolumen des Prismas.

Im Folgenden werden die Merkmale des Drei-Reflexionsflächen-Prismas 112 aufgeführt: Das Drei-Reflexionsflächen-Prisma 112 weist drei Reflexionsflächen 13, 14 und 15 auf, deren Anordnung ähnlich der Anordnung der Reflexionsflächen des Drei-Reflexionsflächen-Prismas 111 ist. Für eine maximale N.A. trifft die Normale N_m der Reflexionsfläche 14, welche die Gerade m enthält, die Kante der beiden anderen Reflexionsflächen 13 und 15 im Punkt B. Dort besteht ein stumpfer Winkel. Ein Randstrahl breitet sich näherungsweise parallel zu Fläche 14 aus. Der Bündelfokus BF liegt zumindest näherungsweise auf der Fläche 14. Der Winkel BAC und der Winkel BCE betragen weniger als 90° (Altgrad) und der Winkel CBD beträgt mehr als 90° (Altgrad). Die Gerade m, welche die Strecke AC enthält, und ein Randstrahl RS im Prisma schließen einen Winkel gamma von weniger als 5° (Altgrad), beispielsweise ≈ 0°, ein. Bevorzugt schließen die Gerade m und ein Randstrahl RS im Prisma einen Winkel kleiner als 1° (Altgrad) ein. Der vorzeichenbehaftete Winkel tau zwischen dem Eingangshauptstrahl EHS und der Reflexionsfläche 14 ist vorzugsweise kleiner als -1° (Altgrad), z.B. zwischen -2° (Altgrad) und -16° (Altgrad). Fertigungstechnisch ist ein Querversatz delta_q von 0,2 mm noch gut zu realisieren. Der halbe Aperturwinkel Alpha_p des Drei-Reflexionsflächen-Prismas 111 beträgt in diesem Beispiel ±15°.

Das Drei-Reflexionsflächen-Prisma 112 ist gut mit einem Mikroskop-Objektiv der Vergrößerung 20x und der N.A. = 0,4 als Referenzreflektor zu kombinieren. Dies erfolgt in Verbindung mit einem Michelson-Interferometer in einem zweiten Ausführungsbeispiel (2) ohne Figur. Dazu wird linear polarisiertes Licht mit senkrechter Polarisationsrichtung verwendet.

Die **Figur 4** stellt eine Mirau-Interferometer-Anordnung zum Messen eines feinbearbeiteten Metallobjekts 107 mit einem Drei-Reflexionsflächen-Prisma 112 mit Strahlenbündelkreuzung dar. Das Drei-Reflexionsflächen-Prisma 112 ist auf die Frontlinse des Mikroskop-Objektivs 51 aufgekittet. Das Mikroskop-Objektiv 51 besitzt eine Brennweite f'= 7 mm und eine numerische Apertur N.A. = 0,5. Der freie Arbeitsabstand zum Metallobjekt 107 beträgt 1,5 mm. Die Mirau-Interferometer-Anordnung ist zwecks Fokussierung in z-Richtung beweglich ausgebildet.

Zwecks Reduzierung von Dispersionseffekten besteht das Strahlteiler-Substrat 72, welches in seiner Dicke von 1,3 mm dem Glasweg des Drei-Reflexionsflächen-Prismas 112 gut angepasst ist, vorzugsweise aus gleichem Material wie das Drei-Reflexionsflächen-Prisma 112. Dies führt zu einem balancierten Interferometer. Im Objektraum ist hier ein außeraxial liegender Messpunkt P' und im Referenzarm ist ein Referenzpunkt P'' dargestellt. Die Position des entfalteten kohärenten Referenzpunktes P''_entf' ist ebenfalls außeraxial und der Punkt P''_entf' steht im gleichen Abstand von der optisches Achse des Mikroskop-Obj ektivs 51 wie der Messpunkt P'. Die so gegebene Symmetrie-Lage im Feld reduziert Fehlereinflüsse. Bei dieser Anordnung wird das räumliche Interferogramm in der Pupille durch eine Zylinderoptik in der Transferstufe komprimiert, so dass die hier durch Abschattung fehlende Mitte in der Pupille kein Problem darstellt. Das **Detail 4** zeigt das Drei-Reflexionsflächen-Prisma 112 vergrößert in Einbaulage.

Die **Figur 5** stellt eine beispielhafte interferometrische Anordnung mit einem Linnik-Interferometer für einen Messpunkt und mit einem Drei-Reflexionsflächen-Prisma 112, sowie einem chromatisch-konfokalen Mess-System in einer gröberen Skala dar, dessen Strahlengang koaxial zum Objektstrahlengang im Objektarm O des Interferometers angeordnet ist. Bei einem Linnik-Interferometer besteht keine Abschattung in der Pupille, was einen Vorteil darstellt. Das hochaperturige Mess-Mikroskop-Objektiv 171 im Strahlengang des Linnik-Interferometers weist eine Brennweite von 7 mm auf. Die N.A. beträgt 0,65 und der Querversatz beträgt delta_q=200 μm. Auf der Frontlinse des Referenzobjektivs 172 ist ein optisch dünnes Apodisations-Filter 222 mit radialsymmetrischer Gauß-Charakteristik angeordnet. Das Apodisations-Filter 222 ermöglicht, die Referenzbündel-Kugelwelle in der N.A. deutlich zu verkleinern, ohne stärkere schädliche Beugungseffekte hervorzurufen, damit die resultierende N.A. dann zur deutlich kleineren N.A. von etwa 0,48 des Drei-Reflexionsflächen-Prismas 112 mit Kreuzstrahlengang passt. Die kleinere NA stellt aber kein Problem dar, da nur in der Pupillenmitte räumliche Interferogramme ausgewertet werden. Das **Detail 5** zeigt das vergrößerte Drei-Reflexionsflächen-Prisma 112 in Einbaulage. Des Weiteren ist ein Beobachtungssystem für das Objekt 108 mit einer Lichtquelle 35 und einer schnellen Matrix-Kamera 209 angeordnet, dessen Strahlengang über die Farb-Teilerwürfel 74 und 73 verknüpft ist. Zusätzlich ist ein chromatisch-konfokales Messsystem für die Gewinnung eines Fokussignals angeordnet, welches mit einer Lichtquelle 31 und einem Detektor 38 der aus einer 100 KHz-Linien-Kamera mit vorgeordnetem Spektrometer für einen Lichtpunkt im Wellenlängenbereich von 580 nm bis 700 nm aufgebaut ist.

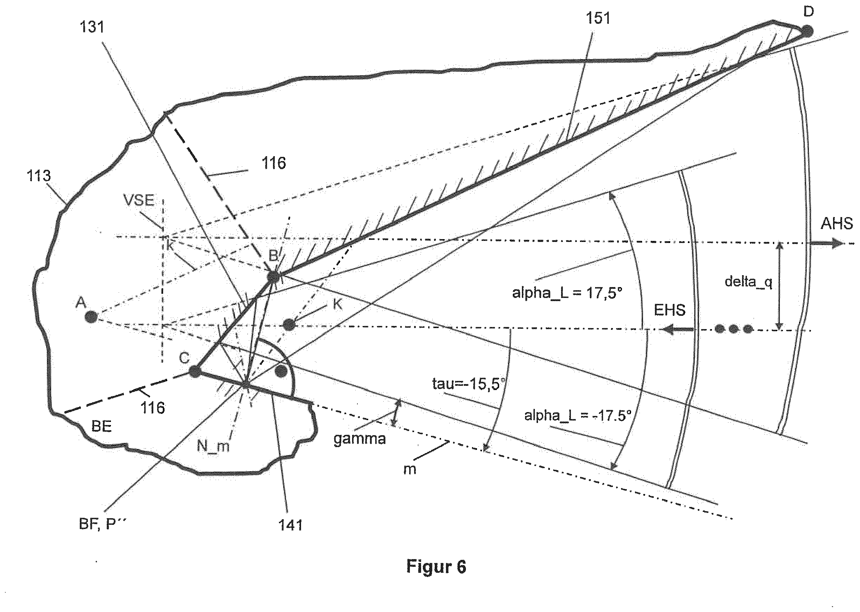

In der **Figur 6** ist eine Drei-Reflexionsflächen-Anordnung 113 (Dreifach-Reflexions-Anordnung) in Luftausführung mit gekreuztem Strahlengang mit Kreuzungspunkt K dargestellt. Diese Anordnung findet Anwendung, wenn ein besonders geringer Versatz delta_q erreicht werden soll. Die Anordnung ist z.B. als Metall-Fügegruppe herstellbar. Diese Anordnung kann für ein Linnik-, ein Mirau- oder ein Michelson-Interferometer als Referenzreflektor von Vorteil sein. Beispielsweise kann die Drei-Reflexionsflächen-Anordnung 113 als Referenzreflektor (Endreflektor im Referenzstrahlengang) in den in Figuren 1, 4 und 5 eingesetzt werden. Bevorzugt wird linear polarisiertes Licht verwendet.

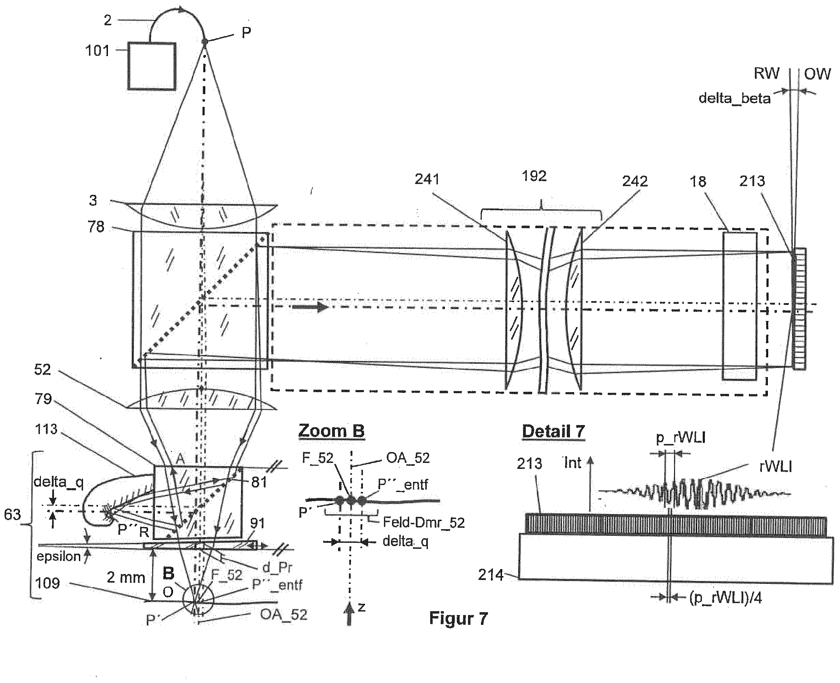

Die **Figur 7** zeigt eine weitere beispielhafte interferometrische Anordnung mit einem Michelson-Interferometer. Das von einer fasergekoppelten Punktlichtquelle 101 mit mehreren Doppel-Superlumineszenz-Dioden im spektralen Bereich von 490 nm bis 680 nm ausgehende Licht passiert den Kollimator 3 und gelangt durch den Ein- und Auskoppel-Strahlteilerwürfel 78 über ein Objektiv 52 (z.B. vom Typ 20x-Objektiv G Plan APO von der Firma Mitutuo mit der Brennweite f'=10 mm und der N.A.=0,28) in das Michelson-Interferometer 63. Das fokussierte Licht trifft auf den Teilerwürfel 71 mit 3,5mm Kantenlänge, was der Deckglas-Korrektur dieses Objektivs entspricht. Dies ergibt einen 2 mm freien Arbeitsabstand zum Messobjekt 109, das z.B. eine Aluminiumoberfläche im Übergangsbereich zwischen optisch glatt und rau darstellt. Die Drei-Reflexionsflächen-Anordnung (Dreifach-Reflexions-Anordnung) 113 in Luftausführung mit einem gekreuzten Strahlengang mit dem Kreuzungspunkt K ist mit einem Querversatz delta_q von 300 μm aufgebaut. **Zoom B** zeigt die symmetrische Nutzung des Feldes des Objektivs 52, da sich die Punkte P' und P''entf in etwa gleichem Abstand vom Brennpunkt F_52 des Objektivs 52 befinden. Aufgrund der vergleichsweise geringen numerischen Apertur ist diese Anordnung eher nicht für die Messung der Rauheit zu verwenden. Hierbei geht es um die Profilmessung. Dazu kommt ein sehr schneller Mehrfach-Fotodioden-Detektor 213 mit Hardware-Prozessor 214 zur Anwendung, um die Lage des Schwerpunktes eines Weißlicht-Interferogramms im Sub-Millisekunden-Bereich zu bestimmen. Um eine numerisch einfache Auswertung zu ermöglichen, beträgt die Periodenlänge p_rWLI einer Oszillation im räumlichen Interferogramm das Vierfache des Pitches der Dioden im Mehrfach-Fotodioden-Detektor 213, was im **Detail 7** dargestellt ist. Dies kann aufgrund des faktisch invarianten Winkels zwischen den interferierenden Wellenfronten RW und OW bei der Detektion delta beta gut eingehalten werden.

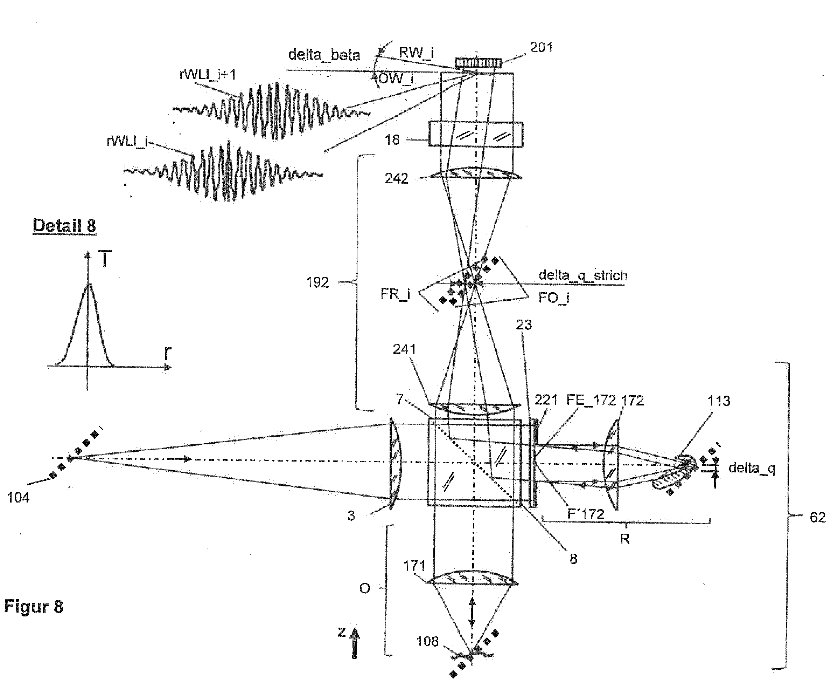

Die **Figur 8** zeigt eine beispielhafte interferometrische Anordnung mit einem Linnik-Interferometer 62 mit einer Multipunkt-Lichtquelle 104, aufgebaut aus mehreren Superlumineszenz-Dioden, und mit einem Mikroskop-Objektiv 171 im Objektarm O des Interferometers 62. Das Mikroskop-Objektiv 171 hat eine numerische Apertur N.A. = 0,5 und eine Brennweite von 6 mm. Das Objekt 108 weist eine raue Oberfläche auf. Im Referenzstrahlengang R des Interferometers 62 ist ein Referenz-Mikroskop-Objektiv 172 mit einer numerischen Apertur N.A. von 0,28 und eine Drei-Reflexions-Anordnung 113 nach **Figur 6** mit gekreuztem Strahlengang angeordnet, deren Querversatz delta_q=250μm beträgt. Es wird eine Vielzahl von Messpunkten gemessen. Zur Anpassung der Drei-Reflexions-Anordnung 113 mit der zum Objektstrahlengang geringeren numerischen Apertur ist ein optisch dünnes Apodisations-Filter 221 mit radialsymmetrischer Gauß-Charakteristik auf einem Substrat 23 in der Fourier-Ebene FE_172 des Referenzobjektivs 172 angeordnet. Die Gauß-Charakteristik des Apodisations-Filters 221 ist im **Detail 8** dargestellt. Der Apodisations-Filter 221 verkleinert das Referenzbündel in der N.A., so dass es zur N.A. von 0,28 der Drei-Reflexions-Anordnung 113 passt. Nach Vereinigung der interferierenden Bündel von Referenz- und Objektstrahlengang mittels Strahlteilerwürfel 7 passieren diese die optische 4f-Transferstufe 192, bestehend aus Tubusobjektiv 241 und Kameraobjektiv 241, und die Zylinderoptik 18. Jeder optisch im Objektstrahlengang anmessbare Messpunkt erzeugt auf der Matrix-Kamera 201 ein Weißlicht-Interferogramm, so dass eine Vielzahl nebeneinander liegender räumlicher Weißlicht-Interferogramme (rWLI_i, wobei i eine Ganzzahl ist) mit gleicher Ortsfrequenz detektiert werden kann. Es ist aber auch möglich, in einem weiteren Ausführungsbeispiel (3) ohne Figur in den beiden Interferometerarmen jeweils eine hier nicht dargestellte 4f-Transferstufe einzusetzen. Dies ist von Vorteil, wenn die Fourier-Ebene des Objektivs 172 durch die Objektivbauweise geometrisch nicht zugänglich ist. So kann das Apodisations-Filter in einer zur Fourier-Ebene FE_172 des Referenzobjektivs 172 optisch konjugierten Ebene angeordnet sein.

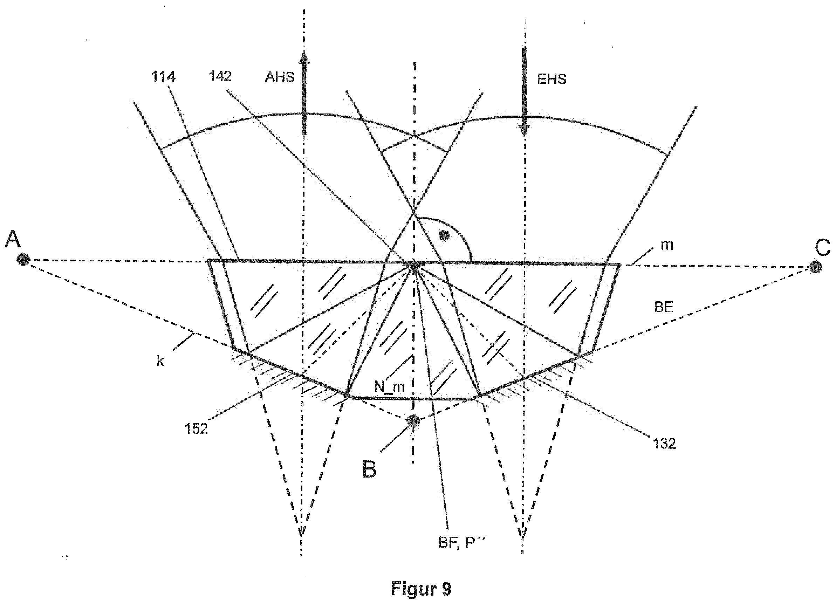

Die **Figur 9** zeigt ein nicht beanspruchtes Drei-Reflexionsflächen-Prisma in W-Ausbildung 114 (d.h. mit einem W-Strahlengang) im Referenzarm eines Zweistrahl-Interferometers, wobei das Prisma eine große numerische Apertur bis zu 0,8 für den Strahlengang im Referenzarm ermöglicht. Die drei optisch wirksamen Flächen 132, 142 und 152 (d.h. Reflexionsflächen) des Drei-Reflexionsflächen-Prismas in W-Ausbildung 114 liegen jeweils zumindest näherungsweise senkrecht zu einer gemeinsamen Bezugsebene BE. Die Reflexionsflächen 132, 142 und 152 können als Planspiegel ausgebildet werden. Die drei Spurgeraden der Ebenen, welche durch die drei Reflexionsflächen 132, 142 und 152 dargestellt werden, bilden in der Bezugsebene BE ein Dreieck ABC mit einem stumpfen Winkel. In dem dargestellten Beispiel liegt die Reflexionsfläche 132 auf einer (verlängerten) Geraden, welche die Punkte C und B verbindet, die Reflexionsfläche 142 auf einer (verlängerten) Geraden m, auf welcher die Punkte A und C liegen, und die Reflexionsfläche 152 auf einer (verlängerten) Geraden k liegt, auf welcher die Punkte A und B liegen. Die Reflexionsfläche 142 und die Reflexionsfläche 132 sind in einem spitzen Winkel relativ zueinander angeordnet (d.h. der Winkel zwischen den Geraden 1 und m ist ein spitzer Winkel). Die Reflexionsfläche 132 und die Reflexionsfläche 152 sind in einem stumpfen Winkel relativ zueinander angeordnet (d.h. der Winkel zwischen den Geraden k und 1 ist ein stumpfer Winkel). Die Reflexionsfläche 142 wird als zweite der drei Reflexionsflächen zur Reflexion eines fokussierten Bündels FB genutzt, die Reflexionsfläche 132 wird als erste oder dritte der drei Reflexionsflächen zur Reflexion genutzt.

Das Drei-Reflexionsflächen-Prisma 114 erzeugt für das Referenzbündel einen Querversatz vom Betrag delta_q, der in Bezug auf die laterale Ausdehnung dieses Prismas besonders groß ist. Ein spezielles Merkmal besteht hierbei darin, dass die Normale N_m vom Punkt B, gelotet auf die Reflexionsfläche 142, welche die Strecke AC enthält, zumindest näherungsweise den Punkt B trifft. Das Drei-Reflexionsflächen-Prisma 114 wird in derselben Weise wie eine Drei-Reflexions-Anordnung mit gekreuztem Strahlengang mit Kreuzungspunkt K genutzt. In einem weiteren Ausführungsbeispiel (4) ohne Figur ist eine Drei-Reflexions-Anordnung mit einem Prisma mit zwei Spiegelflächen und einer totalreflektierenden Fläche ausgebildet, welche die Gerade m enthält. Wichtig ist nur, dass stets drei Reflexionen auftreten oder eine ungeradzahlige Anzahl von Reflexionen im Referenzstrahlengang auftritt. Das für ein Drei-Reflexionsflächen-Prisma 114 in W-Ausbildung zur Anwendung kommende Zweistrahl-Interferometer nach **Figur 9** kann ein Michelson-, ein Mirau- oder auch ein Linnik-Interferometer sein. Die Weglänge des refraktiven Materials dieses Drei-Reflexionsflächen-Prismas 114 wird im Objektarm zur Kompensation eingebracht. In einem weiteren Ausführungsbeispiel (5) ohne Figur kann das Drei-Reflexionsflächen-Prisma 114 auch etwas langgezogen ausgebildet sein, um mehrere Messpunkte entlang einer Linie oder auch eine Linie als Referenzlicht zu ermöglichen.

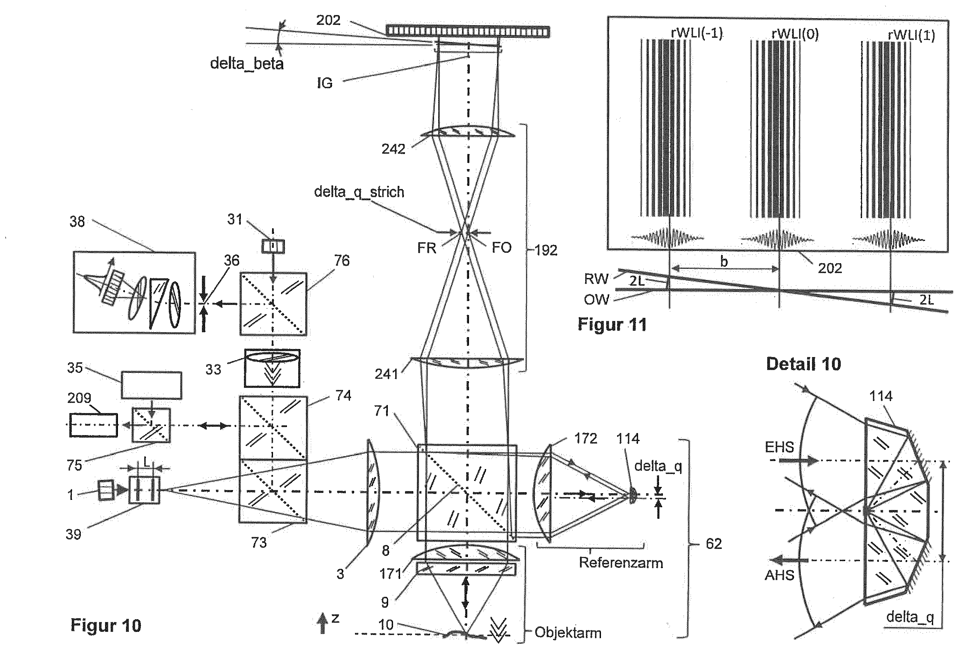

Die Figur 10 stellt ein nicht beanspruchtes Linnik-Interferometer mit einem Drei-Reflexionsflächen-Prisma in W-Ausbildung 114 für einen Messpunkt dar. **Detail 10** zeigt das Drei-Reflexionsflächen-Prisma 114 in Einbaulage. Der fasergekoppelten Punktlichtquelle 1 ist ein faserbasierter Mikroresonator 39, ausgebildet als Fabry-Perot-Interferometer, mit der einfachen optischen Weglänge L des Resonators nachgeordnet, die etwas oberhalb von 10 μm liegt. Es entstehen auch Pulse, die um 2L verzögert sind. Somit ist eine Kohärenzfunktion mit wiederkehrenden Maxima gegeben und so können auch bei einem Gangunterschied von 2L Weißlicht-Interferenzen auftreten. Dies stellt die **Figur 11** dar. Diese Weißlicht-Interferenzen werden mittels schneller Matrixkamera 202 detektiert. Fig. 11 zeigt drei beispielhaften räumlichen Kurzkohärenz-Interferogramme rWLIi, wobei i=0, +1 und -1.

Die Mikroskopobjektive 171 und 172 weisen eine Brennweite von 4 mm und eine numerische Apertur von N.A. = 0,8 auf. Der Querversatz beträgt delta_q=150μm. Auch hier ist wie in der Anordnung gemäß Figur 5 ein Beobachtungssystem für das Objekt 108 mit einer Lichtquelle 35 und einer schnellen Matrix- Kamera 209 angeordnet, dessen Strahlengang über die Farb-Teilerwürfel 74 und 73 verknüpft ist. Und auch hier ist ein chromatisch-konfokales Messsystem für die Gewinnung eines Fokussignals angeordnet, welches mit einer Lichtquelle 31 und einem Detektor 38 der aus einer 100 KHz-Linien-Kamera mit vorgeordnetem Spektrometer für einen Lichtpunkt aufgebaut ist. In einem weiteren Ausführungsbeispiel (6) ohne Figur ist ein Frequenzkamm-Laser mit Mikroresonator als Lichtquelle eingesetzt.

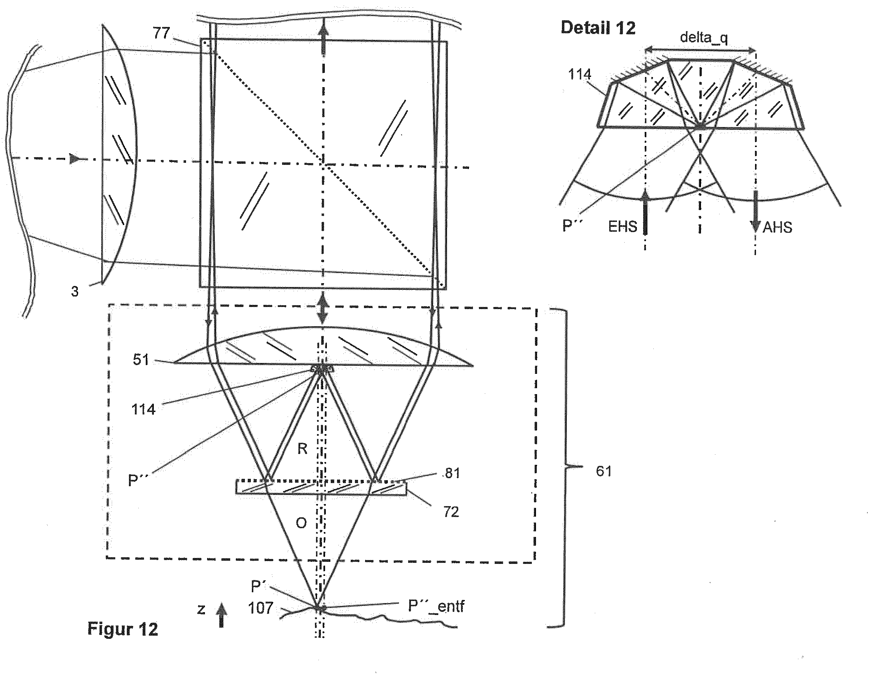

Die **Figur 12** stellt ein nicht beanspruchtes Mirau-Interferometer mit einem Drei-Reflexionsflächen-Prisma in W-Ausbildung 114 für einen Messpunkt dar. Die Brennweite des Mikroskop-Objektiv 51 für das Mirau-Interferometer beträgt 7 mm und die numerische Apertur N.A. 0,65 sowie der Querversatz delta_q=200μm. Auch hierbei wird ein chromatisch-konfokales Mess-System in einer gröberen Skala gemäß Figur 5 zur Fokuslagenregelung eingesetzt, dessen Strahlengang koaxial zum Objekt- Strahlengang im Objektarm O des Interferometers angeordnet ist. Es gibt für das Mirau-Interferometer zwar einen fehlenden Bereich wegen Abschattung aufgrund des Reflexionsprismas in der Pupille. Deshalb ist im ausgehenden Strahlengang vom Mirau-Interferometer eine Zylinderoptik 18 angeordnet, die auf einen linienhaften/schmalen Bereich auf dem Detektor fokussiert. Das **Detail 12** zeigt das vergrößerte Drei-Reflexionsflächen-Prisma 114 in W-Ausbildung in Einbaulage.

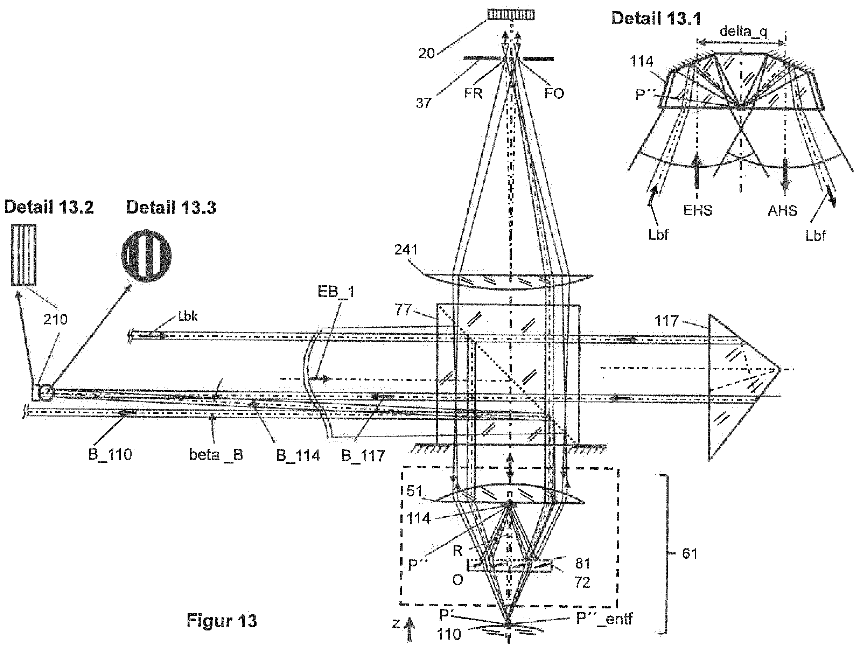

Die **Figur 13** stellt ein nicht beanspruchtes Laserinterferometer 41 zur optischen Antastung des Drei-Reflexionsflächen-Prismas 114 dar, welches sich im Mirau-Objektiv 61 befindet, um eine Bewegung des Mirau-Objektivs 61 in z-Richtung höchstgenau messen zu können.

Der Vorteil durch Nutzung eines Drei-Reflexionsflächen-Prismas 114 besteht darin, dass durch den Winkel beta_B zwischen den 114- und 110-Bündeln eine sehr einfache geometrische Entkopplung vom Laserlicht des Bündels B_110 möglich ist, welches am Objekt 110 direkt reflektiert wird - auch, wenn dies spiegelnd ist. Das vom Objekt 110, hier eine optisch polierte Oberfläche, reflektierte Laserlicht - in Form des Bündels B_110 - wird somit vom Vierfach-Fotodioden-Detektor 210 ferngehalten, um störende Interferenzen zu vermeiden.. Das **Detail 13.1** zeigt das vergrößerte Drei-Reflexionsflächen-Prisma 114 in W-Ausbildung in Einbaulage. Das **Detail 13.2** stellt den Fotodioden-Block 210 und das **Detail 13.3** die dort entstehenden Interferenzen der interferierenden Laserbündel dar.

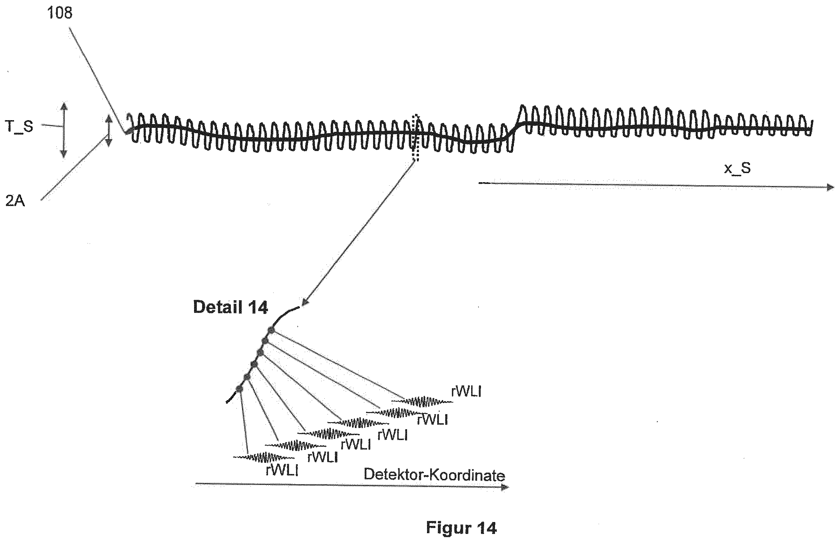

In der **Figur 14** ist eine Möglichkeit dargestellt, auch an Oberflächen Profil und Welligkeit zu messen, deren Profiltiefe die der wellenoptischen Schärfentiefe der interferometrischen Sensor-Anordnung übersteigt. Die Sensor-Anordnung wird dazu schwingend in z-Richtung bewegt und dabei adaptiv den Oberflächenwellen und auch Stufen in der Objektoberfläche mittels eines koaxialen chromatisch konfokalen Sensor-Systems nachgeregelt, s. a. Figur 5. Durch Auslesung einer schnellen Zeilenkamera können räumliche Weißlicht-Interferogramme rWLI detektiert werden. Die Verschiebung in z-Richtung wird permanent hochaufgelöst auch mittels Laserinterferometer 41 gemessen und verrechnet.

Das **Detail 14** zeigt im Zoom Messpunkte auf einer Flanke und die bei einem Tiefen-Scan sich in der Detektorebene dann lateral verschiebende Weißlicht-Interferogramme rWLI. Dabei wurden die Intensitäts-Wavelets aus Gründen der besseren Darstellbarkeit in der Höhe etwas versetzt dargestellt.

Die oben beschriebenen Anordnungen lassen sich grundsätzlich mit allen Zweistrahl-Interferometer realisieren. Die Anordnungen mit einem Mirau-Interferometer können jedoch gewissen technischen Einschränkungen unterliegen, da eine Abschattung in der Pupillenfläche auftreten kann. Durch das Einführen moderater Asymmetrien im Mirau-Interferometer System - in den optischen Weglängen - kann jedoch wie oben beschrieben zumindest eine einseitige Detektion des Kurz-Kohärenz-Interferogramms erfolgen. Die Anordnungen mit einem Michelson oder einem Linnik Interferometer sind frei von diesen Beschränkungen.

Die oben beschriebenen Anordnungen und Verfahren eignen sich zur Erfassung von Abstand, Profil, Form, Welligkeit Rauheit oder der optischen Weglänge in oder an optisch rauen oder glatten Objekten und/oder zur optischen Kohärenz-Tomografie (OCT). Beispielsweise können die oben beschriebenen Anordnungen und Verfahren zur Messungen an gespannten Maschinenbau-Oberflächen mit hoher lateraler und hoher Tiefen-Auflösung im Fertigungsprozess oder in fertigungsnaher Umgebung eingesetzt werden. Ferner ist eine robuste 3D-Erfassung mittels Single-Shot Interferometrie mit einer vergleichsweise hohen numerischen Apertur (bis z.B. N.A. = 0,8 im Extremfall, typischerweise zwischen 0,25 und 0,55) bei einem hohen Brechungsindex der Komponenten möglich. Eine hohe numerische Apertur dient der besseren lateralen Auflösung, um auch an gespannten Maschinenbau-Oberflächen mit hoher lateraler und hoher Tiefen-Auflösung messen zu können, sowie der besseren Lichtausbeute.

**Bezugszeichenliste**

| Bezugszeichen | Bezeichnung |
|---|---|
| 1 | Lichtquelle, z.B. fasergekoppelte Punktlichtquelle mit drei Doppel-Superlumineszenz-Dioden, um die Lichtquelle im spektralen Bereich von 750 nm bis 950 nm zur interferometrischen Nutzung zu betreiben |
| 101 | Lichtquelle, z.B. fasergekoppelte Punktlichtquelle mit mehreren Doppel-Superlumineszenz-Dioden, um die Lichtquelle im spektralen Bereich von 490 nm bis 680 nm zur interferometrischen Nutzung zu betreiben |
| 103 | Lichtquelle für flächenhafte Beleuchtung des Messobjekts, z.B. zur Aufnahme mittels einer Matrix-Kamera |
| 104 | Linienlichtquelle, z.B. aufgebaut aus mehreren Superlumineszenz-Dioden |
| 2 | Single-mode Faser |
| 3 | Kollimator |
| 4 | Sensorkopf, vorzugsweise beweglich präzisionsgeführt in z-Richtung zur hochdynamischen Nachfokussierung |
| 5 | Mikroskop-Objektiv, vorzugsweise mit einer Brennweite $f' = 40$ mm, einer numerische Apertur N.A.=0,09 und mit einem Pupillendurchmesser $d\_P$ in der Fourier-Ebene von 7,2 mm |
| 51 | Mikroskop-Objektiv für Mirau-Interferometer |
| 52 | Mikroskop-Objektiv, z.B. 20x-Mitutuo-Objektiv G Plan APO mit einer Brennweite $f'=10$ mm, einer numerischen Apertur N.A.=0,28, einer einkorrigierten Glasweglänge von 3,5 mm und einem Objektfeld-Durchmesser von 0,55 mm, wozu ein Querversatz delta\_q = 0,3 mm gut passt |
| 6 | Michelson-Interferometer, nutzt den Ausgang B |
| 61 | Mirau- Interferometer |
| 62 | Linnik-Interferometer |
| 63 | Michelson-Interferometer, nutzt den Ausgang A |
| 7 | Strahlteileranordnung, z.B. 112°-Strahlteileranordnung |
| 71 | 90°-Strahlteilerwürfel im Michelson-Interferometer 601 oder Linnik-Interferometer 62 |
| 72 | Substrat für Strahlteiler |
| 73 | Ein- und Auskoppel-Strahlteilerwürfel, welcher vorzugsweise Licht oberhalb 750nm Wellenlänge transmittiert und Licht unterhalb von 720nm reflektiert |
| 74 | Farb-Strahlteiler für Ein- und Auskoppeln von Licht für Kamerabeobachtung, vorzugsweise Transmission oberhalb 560 nm, Reflexion unterhalb 560 nm |
| 75 | 50:50-Ein- und Auskoppel-Strahlteilerwürfel für Beobachtungskamera |
| 76 | 50:50-Ein- und Auskoppel-Strahlteilerwürfel für chromatisch-konfokale Sensorik zur Fokuslagenbestimmung |
| 77 | 50:50-Ein- und Auskoppel-Strahlteilerwürfel für Mirau-Interferometer 61 |
| 78 | 50:50-Ein- und Auskoppel-Strahlteilerwürfel für Michelson-Interferometer 63 |
| 79 | 50:50-Strahlteilerwürfel, z.B. mit 3,5 mm Kantenlänge im Michelson-Interferometer 63 |
| 8 | 50:50-Strahlteiler-Schicht |
| 81 | Strahlteilerschicht, z.B. mit 80% Transmission und 20% Reflexion |
| 91 | Glaskeil |

(fortgesetzt)

| Bezugszeichen | Bezeichnung |
|---|---|
| | Dieser Glaskeil entspricht in der optischen Wirkung auch einer Kompensationsplatte. Dieser Glaskeil ist mit seiner Außenseite senkrecht zur optischen Achse des Mikroskop-Objektivs ausgerichtet. Dieser Glaskeil hat auch eine Schutzfunktion. In Figur 1 beträgt dessen Dicke in der Mitte zwischen 1,4 mm und 1,7 mm - je nach Justierzustand. |
| 93 | Kompensationsplatte in einem Linnik-Interferometer 62. Diese Kompensationsplatte ist hinsichtlich der Glasweglänge auf die Glasweglänge des Drei-Reflexionsflächen-Prismas 112 abgestimmt. |
| 94 | mechanischer Schutzwinkel |
| 10 | Dünnglas als Messobjekt mit 300 $\mu$m Dicke |
| 107 | feinbearbeitetes Metallobjekt |
| 108 | Messobjekt mit rauer Oberfläche |
| 109 | Aluminiumoberfläche im Übergangsbereich zwischen optisch glatt und rau |
| 110 | optisch polierte Oberfläche |
| 111 | Drei-Reflexionsflächen-Prisma mit gekreuztem Strahlengang mit Kreuzungspunkt K mit einem Lateral-Versatz delta_q. In Figur 1 beträgt delta_q = 0,5 mm und die optische Weglänge beträgt um 1,6 mm. |
| 112 | Drei-Reflexionsflächen-Prisma aus Glas mit gekreuztem Strahlengang mit Kreuzungspunkt K und Geometrie für maximale numerische Apertur |
| 113 | Drei-Reflexionsflächen-Anordnung in Luftausführung mit gekreuztem Strahlengang mit Kreuzungspunkt K |
| 114 | Drei-Reflexionsflächen-Prisma in W-Ausbildung |
| 115 | Befestigungskomponenten für Drei-Reflexionsflächen-Prisma 112 |
| 116 | Fügeflächen für Drei-Reflexions-Anordnung in Luftausführung 113 |
| 117 | Tripelprisma, Raumecke |
| 12 | Eingangsfläche des Drei-Reflexionsflächen-Prismas mit gekreuztem Strahlengang |
| 13 | erste Spiegelfläche des Drei-Reflexionsflächen-Prismas 111 |
| 131 | erste Spiegelfläche der Drei-Reflexions-Anordnung in Luftausführung 112 |
| 132 | erste Spiegelfläche des Drei-Reflexionsflächen-Prismas in W-Ausbildung 114 |
| 14 | zweite Spiegelfläche des Drei-Reflexionsflächen-Prismas 111 |
| 141 | zweite Spiegelfläche der Drei-Reflexions-Anordnung in Luftausführung 112 |
| 142 | zweite Spiegelfläche des Drei-Reflexionsflächen-Prismas in W-Ausbildung 114, z.B. als Mikrospiegel ausgebildet |
| 15 | dritte Spiegelfläche des Drei-Reflexionsflächen-Prismas 111 |
| 151 | dritte Spiegelfläche der Drei-Reflexions-Anordnung in Luftausführung 112 |
| 152 | dritte Spiegelfläche des Drei-Reflexionsflächen-Prismas in W-Ausbildung 114 |
| 16 | Ausgangs-Abbildungsstufe Chromatisch voll auskorrigiertes optisches Gesamtsystem 2 vom Interferometer-Ausgang bis zum Detektor |
| 17 | Objektiv am Ausgang des Michelson-Interferometers, f'= 40 mm und N.A. = 0,09 in Figur 1 |
| 171 | hochaperturiges Mess-Mikroskop-Objektiv im Strahlengang eines Linnik-Interferometers |
| 172 | hochaperturiges Referenz-Mikroskop-Objektiv im Strahlengang eines Linnik-Interferometers |
| 18 | Zylinder-Optik zur Verdichtung des Strahlenbündels auf eine schnelle Zeilen-Kamera 20 |

(fortgesetzt)

| Bezugszeichen | Bezeichnung |
|---|---|
| 19 | Auskoppelstrahlteiler-Platte |
| 191 | Strahlteilerschicht auf der Auskoppelstrahlteiler-Platte. Die Strahlteilerschicht dämpft vorzugsweise in Transmission etwas die "Seitenbänder" im Spektrum, damit für die Mehrfach-Fotodioden-Detektoren ein WLI-Wavelet mit einer etwas breiteren schwach modulierten Einhüllenden und auf der schnellen Zeilen-Kamera 20 ein WLI-Wavelet mit einer möglichst schmalen Einhüllenden entsteht. |
| 192 | optische 4f-Transferstufe, bestehend aus Tubusobjektiv 241 und Kameraobjektiv 241 |
| 20 | schnelle Zeilen-Kamera mit 1024 Pixeln, detektiert ein räumliches Weißlicht-Interferogramm (rWLI) |
| 201 | schnelle Matrix-Kamera, detektiert mehrere Weißlicht-Interferogramme (rWLI_ i, wobei i eine Ganzzahl ist) von mehreren Messpunkten |
| 202 | schnelle Matrix-Kamera, detektiert mehrere Weißlicht-Interferogramme von einem Messpunkt |
| 209 | (schnelle) Matrix- Kamera, detektiert ein Messobjekt |
| 210 | (sehr schneller) Vierfach-Fotodioden-Detektor für Laserinterferometer 41 |
| 211 | sehr schneller Mehrfach-Fotodioden-Detektor - auf der linken Feldseite zum Detektieren der linken Amplitudenwerte in Echtzeit für Defokus-Signal |
| 212 | (sehr schneller) Mehrfach-Fotodioden-Detektor - auf der rechten Feldseite zum Detektieren der rechten Amplitudenwerte in Echtzeit für Defokus-Signal |
| 213 | (sehr schneller) Mehrfach-Fotodioden-Detektor mit Hardware-Prozessor |
| 214 | Digitaler Signal-Prozessor zur Berechnung des Gravitationsschwerpunktes (CG - Center of Gravity) und den Ort der Phasenlage null am Gravitationsschwerpunkt |
| 216 | Digitaler Signal-Prozessor zur Berechnung der Differenz der Summensignale der Oszillationen des rWLIs unter der Einhüllenden vom linken und rechten Bereich des Feldes |
| 217 | schneller elektronischer Leistungs-Verstärker |
| 218 | Hochdynamisches Piezosteller-System zur Stellung der Tiefenlage mit integriertem elektronischen Leistungs-Verstärker 217 |
| 219 | Hochdynamisches Mess-System zur Messung der Tiefenlage des in z-Richtung beweglichen Sensorkopfes 4 |
| 221 | Dünnschicht-Apodisationsfilter in Fourier-Ebene FE'172 oder in einer zu dieser optisch konjugierten Ebene |
| 222 | Optisch dünnes Apodisationsfilter mit radialsymmetrischer Gauß-Charakteristik auf Frontlinse des Referenz-Mikroskop-Objektivs 172 Das ermöglicht, die Referenzbündel-Kugelwelle deutlich zu verkleinern, ohne stärkere schädliche Beugungseffekte zu bewirken, damit die resultierende die Referenzbündel-Kugelwelle dann zur deutlich kleineren N.A. von etwa 0,48 des Prismen-Drei-Reflexionen-Referenz-Reflektors passt. Das Messobjektiv 172 im Objektstrahlengang O hat dagegen eine NA von 0,8. Dies stellt aber kein Problem dar, da nur in der Pupillenmitte rWLIs ausgewertet werden. |
| 23 | Substrat für das Apodisationsfilter 221 in der Fourier-Ebene FE_ 172 |
| 241 | Tubusobjektiv |
| 242 | Kameraobj ektiv |
| 31 | Breitband Punktlicht- Quelle, vorzugsweise im Spektralbereich von 580nm bis 700 nm. Vorzugsweise liefert die Breitband PunktlichtQuelle t das |
| | Messlicht für einen chromatisch-konfokalen Punkt-Sensor |
| 33 | Hyperchromatisches System zur Tiefenaufspaltung von Foki |

| Bezugszeichen | Bezeichnung |
|---|---|
| 35 | Lichtquelle, z.B. emittierend im Spektralbereich 480 nm bis 550 nm |
| 36 | Pinhole |
| 37 | Doppel-Lochblende, sperrt alle Bündel außer die für das rWLI |
| 38 | Detektor, der aus einer 100 KHz-Linien-Kamera mit vorgeordnetem Spektrometer für einen Lichtpunkt im Wellenlängenbereich von 580 nm bis 700 nm aufgebaut ist |
| 39 | faserbasierter Mikroresonator, ausgebildet als Fabry-Perot-Interferometer |
| 41 | Laserinterferometer um Bewegung des Sensorkopfes in z-Richtung zu messen |
| | |
| A | lokaler Amplitudenwert |
| AHS | Ausgangshauptstrahl des aus dem Endreflektor austretenden Bündels |
| alpha_P | halber Aperturwinkel im Glas des Prismas 112 |
| b | lateraler Abstand zwischen benachbarten rWLI |
| beta_B - | Winkel zwischen den reflektierten Laserstrahl-Bündeln vom Drei-Reflexionsflächen-Prisma 114 und Objekt 110 |
| BF | Bündelfokus |
| BE | Bezugsebene |
| EB 1 | Eingangsbündel von Lichtquelle 1 |
| EHS | Eingangshauptstrahl des in den Endreflektor des eintretenden Bündels |
| epsilon | Keilwinkel des Glaskeils 91, der vorzugsweise kleiner/gleich ein Altgrad ist |
| delta_beta - | Winkel zwischen den interferierenden Wellenfronten RW und OW bei der Detektion |
| delta_q | Querversatz des Bündels im Referenzarm eines Interferometers |
| d P | Pupillendurchmesser des Mikroskop-Objektivs 5 |
| d_ Pr | Mittendicke des Glaskeils 91 |
| EB_1 - | Eingangsbündel von fasergekoppelter Punktlichtquelle 1 kommend, nach Kollimator 3 |
| f'5 | Brennweite des Mikroskop-Objektivs 5 |
| F'52 | Brennpunkt des Mikroskop-Objektivs 52 |
| FE_ 5 | Fourier-Ebene/Brennebene des Mikroskop-Objektivs 5 |
| FE 172 | Fourier-Ebene/Brennebene des Mikroskop-Objektivs 172 |
| Feld-Dmr_52 - | Felddurchmesser des 20x Mitutuo-Mikroskop-Objektivs 52, Dieser beträgt um 0,5 mm. |
| FO | Fokus des Objektstrahlenbündels |
| FO_i | Foki der Objektstrahlenbündel der Anzahl i |
| FR | Fokus des Referenzstrahlenbündels |
| FR_i | Foki der Referenzstrahlenbündel der Anzahl i |
| gamma | Winkel |
| k | Gerade |
| kappa | Winkel zwischen optischer Achse des Objektstrahlengangs im Interferometer 6 und optischer Achse des Strahlengangs am Ausgang B des Interferometers 6 |
| IG | Interferenzgebiet |
| L | einfache optische Weglänge des Mikroresonators |

(fortgesetzt)

| Bezugszeichen | Bezeichnung |
|---|---|
| Lbk | Laserbündel kollimiert |
| Lbf | Laserbündel fokussiert |
| rWLI | räumliches Kurzkohärenz-Interferogramm |
| rWLI_i | räumliche Kurzkohärenz-Interferogramme der Anzahl i |
| OPD | optischer Gangunterschied (optical path difference) |
| N.A. | numerische Apertur |
| OW | Objektwellenfront, geneigt zur Referenz-Wellenfront RW |
| OW | Objekt -Wellenfront |
| P | Leuchtpunkt |
| P' | Bild des Leuchtpunkts im Objektarm |
| P" | Bild des Leuchtpunkts im Referenzarm |
| P"_entf | Bild des optisch konjugierten Leuchtpunkts P" im Objektraum nach Entfaltung, der kohärent ist zu P' |
| r | Radius des Apodisationsfilters 18 |
| p rWLI | Periodenlänge einer Oszillation im räumlichen Interferogramm (rWLI) |
| RAS | Randstrahl des fokussierten Bündels |
| RW | Referenzwellenfront, geneigt zur Objekt-Wellenfront OW |
| T | Transmissionsgrad eines Apodisationsfilters 221 oder 222 über dem Radius r |
| T_S - | Tiefenmessbereich der WLI-Sensor-Anordnung, insbesondere in Form eines Mirau-Interferometers 61 |
| tau | Winkel zwischen Eingangshauptstrahl EHS und Spiegelfläche 14 |
| VSE | Virtuelle Spiegelebene |
| rWLI | räumliches Weißlicht-Interferogramm |
| x O | Vorschubrichtung des Objekts |
| x_S | Vorschubrichtung der Sensor-Anordnung |
| z_rWLI | Tiefenmesswert, welcher aus dem rWLI-Signal errechnet wird |

**Patentansprüche**

1. Anordnung zur robusten Zweistrahl-Interferometrie, welche folgende Mittel umfasst:

   eine Quelle kurzkohärenter elektromagnetischer Strahlung (1,103, 104) zur Beleuchtung eines Objekts (10, 107, 108, 109, 110),
   ein Interferometer mit einem Objektstrahlengang (O), einem Referenzstrahlengang (R) und einer Messebene (ME) im Objektstrahlengang, in der sich zumindest näherungsweise die optisch anzumessenden Oberflächen- oder Volumenelemente des Objekts (10, 107, 108, 109, 110) befinden; und
   mindestens einen gerasterten Detektor (20, 201, 202) zur Detektion elektromagnetischer Strahlung in Form mindestens eines räumlichen Interferogramms,

   wobei:

   im Referenzstrahlengang (R) des Interferometers mindestens ein End-Reflektor als Referenzreflektor angeordnet ist, wobei der End-Reflektor als eine Dreifach-Reflexions-Anordnung (113) mit drei Reflexionsflächen ausgebildet ist,

die drei Reflexionsflächen jeweils zumindest näherungsweise senkrecht zu einer gemeinsamen Bezugsebene (BE) liegen; und

die drei Spurgeraden der Ebenen, welche durch die drei Reflexionsflächen dargestellt werden, in der Bezugsebene (BE) ein Dreieck ABC mit einem stumpfen Winkel bilden, wobei die erste Reflexionsfläche (13, 131, 132) auf einer Geraden liegt, auf welcher die Punkte C und B liegen, die zweite Reflexionsfläche (14, 141, 142) auf einer Geraden (m) liegt, auf welcher die Punkte A und C liegen und die dritte Reflexionsfläche (15, 151, 152) auf einer Geraden (k) liegt, auf welcher die Punkte A und B liegen,

**gekennzeichnet dadurch, dass**:

der Strahlengang der Dreifach-Reflexions-Anordnung gekreuzt ist,

die zweite Reflexionsfläche (14, 141) als zweite der drei Reflexionsflächen zur Reflexion eines fokussierten Bündels FB genutzt wird und die erste Reflexionsfläche (13, 131) als erste oder dritte der drei Reflexionsflächen zur Reflexion genutzt wird,

die erste Reflexionsfläche (13, 131, 132) und die zweite Reflexionsfläche (14, 141, 142) in einem spitzen Winkel relativ zueinander angeordnet sind,

die erste Reflexionsfläche (13, 131) und die dritte Reflexionsfläche (15, 151) in einem stumpfen Winkel CBD relativ zueinander angeordnet sind,

es eine Normale N_m vom Punkt B auf die zweite Reflexionsfläche (14, 141) gibt, und

für die Dreifach-Reflexions-Anordnung (111, 112, 113) ein Winkel gamma zwischen einem Randstrahl (RAS) des ein- oder des austretenden Bündels und der zweiten Reflexionsfläche (14, 141) besteht und dabei für den Winkel gamma < 12° gilt.

2. Anordnung zur robusten Zweistrahl-Interferometrie nach Anspruch 1,
   wobei der Bündelfokus (BF) zumindest näherungsweise auf der Normalen N_m und zumindest näherungsweise in der Nähe der zweiten Reflexionsfläche (14, 141) liegt; und/oder
   wobei die Dreifach-Reflexions-Anordnung (113) als Luftspiegelgruppe oder als Prismenspiegelgruppe ausgebildet ist.

3. Anordnung zur robusten Zweistrahl-Interferometrie nach mindestens einem der vorangegangenen Ansprüche,
   wobei die vom fokussierten Bündel (FB) genutzte dritte Reflexionsfläche (15, 151) mehr als dreifach so lang ausgebildet ist als die zweite Reflexionsfläche (14, 141); und/oder
   wobei die Dreifach-Reflexions-Anordnung (112) mit einem vorzeichenbehafteten Winkel tau kleiner als -1° ausgebildet ist, wobei der Winkel tau der Winkel zwischen einem Eingangshauptstrahl (EHS) des eintretenden Bündels und der zweiten Reflexionsfläche (14, 141) ist.

4. Anordnung zur robusten Zweistrahl-Interferometrie nach mindesten einem der vorangegangenen Ansprüche, wobei die Lichtquelle als Frequenzkamm-Laser mit einer Mikro-Kavität ausgebildet ist.

5. Anordnung zur robusten Zweistrahl-Interferometrie nach mindesten einem der vorangegangenen Ansprüche, wobei es im Objektmessfeld mindestens ein Tiefenmesssystem zum Erfassen des Messobjekts gibt, welches mit seinem Strahlengang zumindest näherungsweise koaxial zum interferometrischen Strahlengang angeordnet ist, wobei das Tiefenmesssystem optional chromatisch-konfokal ausgebildet ist.

6. Anordnung zur robusten Zweistrahl-Interferometrie nach mindestens einem der vorangegangenen Ansprüche, wobei dem Interferometer im Referenzarm ein Abschwächungsfilter (221, 222) mit einem Maximum der Transmission im Zentrum des Abschwächungsfilters zur Verringerung des Aperturwinkels des Referenzstrahlenbündels zugeordnet ist, wobei optional:

   das Abschwächungsfilter (221, 222) mit radialsymmetrischer Gauß-Charakteristik ausgebildet ist; oder
   das Abschwächungsfilter mit einer eindimensionalen Charakteristik ausgebildet ist.

7. Anordnung zur robusten Zweistrahl-Interferometrie nach mindestens einem der vorangegangenen Ansprüche, wobei auf die dritte Reflexionsfläche (15, 151) ein streifender Einfall zumindest für den Randstrahl eines Bündels mit einem Einfallswinkel größer als 75° besteht.

8. Anordnung zur robusten Zweistrahl-Interferometrie nach mindestens einem der vorangegangenen Ansprüche, wobei:

das Interferometer und der Detektor (20, 201, 202) innerhalb eines Sensorkopfes (4) angeordnet sind; und
dem Sensorkopf (4) ein hochdynamisches Piezosteller-System (218) mit diesem beigeordneten hochauflösenden Tiefenmesssystem (219) zugeordnet ist.

9. Anordnung zur robusten Zweistrahl-Interferometrie nach mindestens einem der vorangegangenen Ansprüche, wobei der Winkel kappa zwischen optischer Achse des Objektstrahlengangs (O) im Interferometer und optischer Achse des Strahlengangs am Ausgang des Interferometers zwischen 96° und 140° liegt.

10. Anordnung zur robusten Zweistrahl-Interferometrie nach mindestens einem der vorangegangenen Ansprüche, wobei ein schiebbarer Glaskeil (91) angeordnet ist, wobei ferner optional:
die Dreifach-Reflexions-Anordnung (111, 112) als ein Drei-Reflexionsflächen-Prisma ausgebildet ist; und wobei die Mittendicke d_Pr des Glaskeils (91) zumindest näherungsweise der Glasweglänge eines Drei-Reflexionsflächen-Prismas (111, 112) entspricht.

11. Verfahren zur robusten Zweistrahl-Interferometrie zur Erfassung von Abstand, Tiefe, Profil, Form, Welligkeit und/oder Rauheit oder der optischen Weglänge in oder an technischen oder biologischen Objekten, und/oder zur optischen Kohärenz-Tomografie (OCT) mit Bildung eines räumlichen Weißlicht-Interferogramms, umfassend:

Bereitstellen einer Anordnung zur robusten Zweistrahl-Interferometrie gemäß zumindest einem der vorangegangenen Ansprüche,
wobei im Referenzstrahlengang (R) eine Reduzierung des Aperturwinkels des Referenz-Strahlenbündels mit bündelbegrenzenden Mitteln (221, 222) durchgeführt wird.

12. Verfahren zur robusten Zweistrahl-Interferometrie nach Anspruch 11, wobei die Reduzierung des Aperturwinkels des Referenz-Strahlenbündels mit bündelbegrenzenden Mitteln zumindest näherungsweise in der Fourier-Ebene eines Objektivs im Referenzstrahlengang (R) eines Linnik-Interferometers (62) durchgeführt wird.

13. Verfahren zur robusten Zweistrahl-Interferometrie nach mindestens einem der Ansprüche 11 und 12, umfassend Bilden eines Balance-Signals für eine Fokus-Regelung des Interferometers (6, 61, 62), wobei um das Balance-Signal zu bilden, die Beträge I_links und I_rechts der Intensitäten benachbarter Fotoelemente eines Fotodioden-Detektors (211, 212, 213) oder Pixel eines gerasterten Detektors, die vom räumlichen Weißlicht-Interferogramm (WLI) links und rechts von einem Referenzpunkt RP angeordnet sind, mittels Rechenwerk (214, 216) bestimmt und aufsummiert werden, wodurch jeweils die Summen S_links und S_rechts erhalten werden; und die beiden Summen Slinks und S_rechts subtrahiert werden und daraus das vorzeichenbehaftete Balance-Signal abgeleitet wird.

14. Verfahren zur robusten Zweistrahl-Interferometrie nach mindestens einem der Ansprüche 11 bis 13, wobei das Interferometer und der Detektor (20, 201, 202) innerhalb eines Sensorkopfes (4) angeordnet sind, und dem Sensorkopf (4) elektromechanische hochdynamische Mittel (218) zur Nachführung des Sensorkopfes (4) zugeordnet sind, die in Mess-Echtzeit den Sensorkopf (4) an jedem kooperativen Messpunkt stets im wellenoptischen Schärfentiefebereich halten,
wobei das Messergebnis stets aus der vorzeichenbehafteten Addition des Tiefenmesswertes des Nachführ-Tiefenmesssystems und des Tiefenmesswertes z_rWLI, welcher aus dem räumlichen Weißlicht-Interferogramm errechnet wird, gebildet wird.

15. Verfahren zur robusten Zweistrahl-Interferometrie zur Erfassung von Abstand, Tiefe, Profil, Form, Welligkeit und/oder Rauheit oder der optischen Weglänge in oder an technischen oder biologischen Objekten, und/oder zur optischen Kohärenz-Tomografie (OCT) mit Bildung eines räumlichen Weißlicht-Interferogramms, umfassend:
Bereitstellen einer Anordnung zur robusten Zweistrahl-Interferometrie gemäß einem der Ansprüche 1 bis 10,
wobei eine zweite optische Antastung des End-Reflektors (111, 112, 113, 114) mittels eines zweiten separaten Interferometers mit einer Laserlichtquelle durchgeführt wird, um eine Verschiebung in z-Richtung des zum End-Reflektor (114) gehörenden Interferometers (61) messen zu können, welches mit dem End-Reflektor (111, 112, 113, 114) starr verbunden ist.

**Claims**

1.  An arrangement for robust two-beam interferometry, which comprises the following means:

    a source of short-coherent electromagnetic radiation (1, 103, 104) for illuminating an object (10, 107, 108, 109, 110),
    an interferometer with an object beam path (O), a reference beam path (R) and a measurement plane (ME) in the object beam path, in which the surface or volume elements of the object (10, 107, 108, 109, 110) to be measured are at least approximately located; and
    at least one rasterized detector (20, 201, 202) for detection of electromagnetic radiation in the form of at least one spatial interferogram,
    wherein:

    at least one end reflector is arranged as a reference reflector in the reference beam path (R) of the interferometer, the end reflector being formed as a triple reflection arrangement (113) with three reflection surfaces,
    the three reflection surfaces are each at least approximately perpendicular to a common reference plane (BE); and
    the three traces of the planes, which are represented by the three reflection surfaces, form a triangle ABC with an obtuse angle in the reference plane (BE), the first reflection surface (13, 131, 132) lying on a straight line on which the points C and B lie, the second reflection surface (14, 141, 142) lying on a straight line (m) on which the points A and C lie, and the third reflection surface (15, 151, 152) lying on a straight line (k) on which the points A and B lie,

    **characterized in that**:

    the beam path of the triple reflection arrangement is crossed,
    the second reflection surface (14, 141) is used as the second of the three reflection surfaces for reflection of a focused bundle FB and the first reflection surface (13, 131) is used as the first or third of the three reflection surfaces for reflection,
    the first reflection surface (13, 131, 132) and the second reflection surface (14, 141, 142) are arranged at an acute angle relative to one another,
    the first reflection surface (13, 131) and the third reflection surface (15, 151) are arranged at an obtuse angle CBD relative to one another,
    there is a normal $N\_m$ from point B to the second reflecting surface (14, 141), and
    for the triple reflection arrangement (111, 112, 113) there is an angle gamma between an edge beam (RAS) of the incoming or outgoing bundle and the second reflection surface (14, 141), and for the angle gamma it holds that $<12°$.

2.  The arrangement for robust two-beam interferometry according to claim 1,
    wherein the bundle focus (BF) lies at least approximately on the normal $N\_m$ and at least approximately in the vicinity of the second reflection surface (14, 141); and/or
    the triple reflection arrangement (113) is formed as an air mirror group or as a prism mirror group.

3.  The arrangement for robust two-beam interferometry according to at least one of the preceding claims,
    wherein the third reflection surface (15, 151) used by the focused bundle (FB) is more than three times as long as the second reflection surface (14, 141); and/or
    wherein the triple reflection arrangement (112) is formed with a signed angle tau smaller than -1°, the angle tau being the angle between an input main beam (EHS) of the incoming bundle and the second reflection surface (14, 141).

4.  The arrangement for robust two-beam interferometry according to at least one of the preceding claims, wherein the light source is formed as a frequency comb laser with a micro-cavity.

5.  The arrangement for robust two-beam interferometry according to at least one of the preceding claims, wherein there is at least one depth measuring system for detecting the measurement object in the object measurement field, which is arranged with its beam path at least approximately coaxially with the interferometric beam path, the depth measuring system optionally being formed to be chromatic-confocal.

6. The arrangement for robust two-beam interferometry according to at least one of the preceding claims, wherein an attenuation filter (221, 222) with a maximum of transmission in the center of the attenuation filter is assigned to the interferometer in the reference arm to reduce the aperture angle of the reference beam bundle, wherein optionally:

the attenuation filter (221, 222) is formed with a radially symmetrical Gaussian characteristic; or
the attenuation filter is formed with a one-dimensional characteristic.

7. The arrangement for robust two-beam interferometry according to at least one of the preceding claims, wherein there is a grazing incidence on the third reflection surface (15, 151) at least for the edge beam of a bundle with an angle of incidence of greater than 75°.

8. The arrangement for robust two-beam interferometry according to at least one of the preceding claims, wherein:

the interferometer and the detector (20, 201, 202) are arranged within a sensor head (4); and
the sensor head (4) is assigned a highly dynamic piezo actuator system (218) with an associated high-resolution depth measuring system (219).

9. The arrangement for robust two-beam interferometry according to at least one of the preceding claims, wherein the angle kappa between the optical axis of the object beam path (O) in the interferometer and the optical axis of the beam path at the output of the interferometer is between 96° and 140°.

10. The arrangement for robust two-beam interferometry according to at least one of the preceding claims, wherein a slidable glass wedge (91) is arranged, wherein furthermore optionally:
the triple reflection arrangement (111, 112) is formed as a triple reflection surface prism; and wherein the center thickness d_Pr of the glass wedge (91) corresponds at least approximately to the glass path length of a three-reflection surface prism (111, 112).

11. A method for robust two-beam interferometry for the detection of distance, depth, profile, shape, ripple and/or roughness or the optical path length in or on technical or biological objects, and/or for optical coherence tomography (OCT) with formation of a spatial white light interferogram, comprising:

providing an arrangement for robust two-beam interferometry according to at least one of the preceding claims, wherein in the reference beam path (R) a reduction in the aperture angle of the reference beam bundle is carried out using bundle-limiting means (221, 222).

12. The method for robust two-beam interferometry according to claim 11, wherein the reduction in the aperture angle of the reference beam bundle with bundle-limiting means is carried out at least approximately in the Fourier plane of an objective in the reference beam path (R) of a Linnik interferometer (62).

13. The method for robust two-beam interferometry according to at least one of claims 11 and 12, comprising forming a balance signal for a focus control of the interferometer (6, 61, 62), wherein
in order to form the balance signal, the amounts I_left and I_right of the intensities of adjacent photo elements of a photodiode detector (211, 212, 213) or pixels of a rasterized detector, which from the spatial white light interferogram (WLI) are arranged to the left and right of a reference point RP, are determined by means of an arithmetic unit (214, 216) and are added up, whereby the sums S_left and S_right are obtained in each case; and
the two sums S_left and S_right are subtracted and the signed balance signal is derived therefrom.

14. A method for robust two-beam interferometry according to at least one of claims 11 to 13, wherein the interferometer and the detector (20, 201, 202) are arranged within a sensor head (4), and
the sensor head (4) is assigned electromechanical, highly dynamic means (218) for tracking the sensor head (4), which in measurement real time always keep the sensor head (4) at every cooperative measuring point in the wave-optical depth of field,
wherein the measurement result is always formed from the signed addition of the depth measurement value of the tracking depth measuring system and the depth measurement value z_rWLI, which is calculated from the spatial white light interferogram.

15. A method for robust two-beam interferometry for the detection of distance, depth, profile, shape, ripple and/or roughness or the optical path length in or on technical or biological objects, and/or for optical coherence tomography (OCT) with formation of a spatial white light interferogram, comprising:

> providing an arrangement for robust two-beam interferometry according to one of claims 1 to 10,
> wherein a second optical scanning of the end reflector (111, 112, 113, 114) is carried out by means of a second separate interferometer with a laser light source in order to be able to measure a displacement in the z direction of the interferometer (61) belonging to the end reflector (114), which is rigidly connected to the end reflector (111, 112, 113, 114).

**Revendications**

1. Agencement d'interférométrie robuste à deux faisceaux, qui comprend les moyens suivants :

   une source de rayonnement électromagnétique à cohérence courte (1, 103, 104) pour l'éclairage d'un objet (10, 107, 108, 109, 110),
   un interféromètre avec une trajectoire de rayons d'objet (O), une trajectoire de rayons de référence (R) et un plan de mesure (ME) dans la trajectoire de rayons d'objet dans lequel les éléments de surface ou volume de l'objet (10, 107, 108, 109, 110) à mesurer optiquement se trouvent au moins approximativement ; et au moins un détecteur tramé (20, 201, 202) pour la détection de rayonnement électromagnétique sous forme d'au moins un interférogramme spatial,
   dans lequel :

   au moins un réflecteur terminal est agencé en tant que réflecteur de référence dans la trajectoire de rayons de référence (R) de l'interféromètre, dans lequel le réflecteur terminal est réalisé en tant qu'agencement de réflexion triple (113) avec trois faces de réflexion,
   les trois faces de réflexion se situent chacune au moins approximativement perpendiculairement à un plan de référence commun (BE) ; et
   les trois traces des plans qui sont représentées par les trois faces de réflexion forment un triangle ABC avec un angle obtus dans le plan de référence (BE),
   dans lequel la première face de réflexion (13, 131, 132) se situe sur une droite sur laquelle les points C et B se situent, la deuxième face de réflexion (14, 141, 142) se situe sur une droite (m) sur laquelle les points A et C se situent, et la troisième face de réflexion (15, 151, 152) se situe sur une droite (k) sur laquelle les points A et B se situent,

   **caractérisé en ce que** :

   la trajectoire de rayons de l'agencement de réflexion triple est croisée,
   la deuxième face de réflexion (14, 141) est utilisée en tant que deuxième des trois faces de réflexion pour la réflexion d'un faisceau concentré FB et la première face de réflexion (13, 131) est utilisée en tant que première ou troisième des trois faces de réflexion pour la réflexion,
   la première face de réflexion (13, 131, 132) et la deuxième face de réflexion (14, 141, 142) sont agencées à un angle aigu l'une par rapport à l'autre,
   la première face de réflexion (13, 131) et la troisième face de réflexion (15, 151) sont agencées à un angle obtus CBD l'une par rapport à l'autre,
   il y a une perpendiculaire N_m du point B sur la deuxième face de réflexion (14, 141), et
   pour l'agencement de réflexion triple (111, 112, 113), un angle gamma existe entre un rayon de bord (RAS) du faisceau entrant ou sortant et la deuxième face de réflexion (14, 141) et gamma < 12° est en l'occurrence valable pour l'angle.

2. Agencement d'interférométrie robuste à deux faisceaux selon la revendication 1, dans lequel le foyer de faisceau (BF) se situe au moins approximativement sur la perpendiculaire N_m et au moins approximativement à proximité de la deuxième face de réflexion (14, 141) ; et/ou
   dans lequel l'agencement de réflexion triple (113) est réalisé en tant que groupe de miroirs à air ou en tant que groupe de miroirs à prismes.

3. Agencement d'interférométrie robuste à deux faisceaux selon au moins une des revendications précédentes, dans lequel la troisième face de réflexion (15, 151) utilisée par le faisceau concentré (FB) est réalisée plus de trois fois aussi longue que la deuxième face de réflexion (14, 141) ; et/ou

dans lequel l'agencement de réflexion triple (112) est réalisé avec un angle tau à signe inférieur à -1°, dans lequel l'angle tau est l'angle entre un rayon principal d'entrée (EHS) du faisceau entrant et la deuxième face de réflexion (14, 141).

4. Agencement d'interférométrie robuste à deux faisceaux selon au moins une des revendications précédentes, dans lequel la source lumineuse est réalisée en tant que laser à peigne de fréquence avec une microcavité.

5. Agencement d'interférométrie robuste à deux faisceaux selon au moins une des revendications précédentes, dans lequel il y a dans le champ de mesure d'objet au moins un système de mesure de profondeur pour la détection de l'objet de mesure qui est agencé avec sa trajectoire de rayons au moins approximativement de manière coaxiale à la trajectoire de rayons interférométrique, dans lequel le système de mesure de profondeur est réalisé en option de manière confocale chromatique.

6. Agencement d'interférométrie robuste à deux faisceaux selon au moins une des revendications précédentes, dans lequel un filtre d'affaiblissement (221, 222) avec un maximum de la transmission dans le centre du filtre d'affaiblissement pour la réduction de l'angle d'ouverture du faisceau de rayons de référence est associé à l'interféromètre dans le bras de référence, dans lequel en option :

le filtre d'affaiblissement (221, 222) est réalisé avec une caractéristique de Gauß à symétrie radiale ; ou
le filtre d'affaiblissement est réalisé avec une caractéristique unidimensionnelle.

7. Agencement d'interférométrie robuste à deux faisceaux selon au moins une des revendications précédentes, dans lequel une incidence rasante au moins pour le rayon de bord d'un faisceau avec un angle d'incidence supérieur à 75° existe sur la troisième face de réflexion (15, 151).

8. Agencement d'interférométrie robuste à deux faisceaux selon au moins une des revendications précédentes, dans lequel :

l'interféromètre et le détecteur (20, 201, 202) sont agencés au sein d'une tête de capteur (4) ; et
un système d'actionneur piézoélectrique hautement dynamique (218) avec ce système de mesure de profondeur (219) à haute résolution coordonné est associé à la tête de capteur (4).

9. Agencement d'interférométrie robuste à deux faisceaux selon au moins une des revendications précédentes, dans lequel l'angle kappa entre l'axe optique de la trajectoire de rayons d'objet (O) dans l'interféromètre et l'axe optique de la trajectoire de rayons à la sortie de l'interféromètre se situe entre 96° et 140°.

10. Agencement d'interférométrie robuste à deux faisceaux selon au moins une des revendications précédentes, dans lequel un coin de verre coulissant (91) est agencé, dans lequel en outre en option :
l'agencement de réflexion triple (111, 112) est réalisé en tant que prisme à trois faces de réflexion ; et dans lequel l'épaisseur moyen d_Pr du coin de verre (91) correspond au moins approximativement au trajet optique du verre d'un prisme à trois faces de réflexion (111, 112).

11. Procédé d'interférométrie robuste à deux faisceaux pour la détection d'écart, profondeur, profil, forme, ondulation et/ou rugosité ou du trajet optique dans ou sur des objets techniques ou biologiques, et/ou pour la tomographie en cohérence optique (OCT) avec formation d'un interférogramme à lumière blanche spatial, comprenant :

mise à disposition d'un agencement d'interférométrie robuste à deux faisceaux selon au moins une des revendications précédentes,
dans lequel une réduction de l'angle d'ouverture du faisceau de rayons de référence avec des moyens à limitation de faisceau (221, 222) est réalisée dans la trajectoire de rayons de référence (R).

12. Procédé d'interférométrie robuste à deux faisceaux selon la revendication 11, dans lequel la réduction de l'angle d'ouverture du faisceau de rayons de référence avec des moyens à limitation de faisceau est réalisée au moins approximativement dans le plan de Fourier d'un objectif dans la trajectoire de rayons de référence (R) d'un interféromètre de Linnik (62).

13. Procédé d'interférométrie robuste à deux faisceaux selon au moins une des revendications 11 et 12, comprenant former un signal d'équilibre pour une régulation de foyer de l'interféromètre (6, 61, 62), dans lequel pour former le signal d'équilibre, les montants I_links et I_rechts des intensités de photo-éléments voisins d'un détecteur à photodiodes (211, 212, 213) ou des pixels d'un détecteur tramé qui sont agencés à gauche et à droite d'un point de référence RP par l'interférogramme à lumière blanche spatial (WLI) sont déterminés et additionnés au moyen d'une unité de calcul (214, 216), moyennant quoi les sommes S_links et S_rechts sont à chaque fois obtenues ; et les deux sommes S_links et S_rechts sont soustraites et le signal d'équilibre à signe en est dérivé.

14. Procédé d'interférométrie robuste à deux faisceaux selon au moins une des revendications 11 à 13, dans lequel l'interféromètre et le détecteur (20, 201, 202) sont agencés au sein d'une tête de capteur (4), et des moyens électromécaniques hautement dynamiques (218) pour le guidage précis de la tête de capteur (4) qui maintiennent en temps réel de mesure la tête de capteur (4) à chaque point de mesure coopératif toujours dans la région de profondeur de champ optique ondulatoire sont associés à la tête de capteur (4), dans lequel le résultat de mesure est toujours formé de l'addition à signe de la valeur de mesure de profondeur du système de mesure de profondeur à guidage précis et de la valeur de mesure de profondeur z_rWLI qui est calculée à partir de l'interférogramme à lumière blanche spatial.

15. Procédé d'interférométrie robuste à deux faisceaux pour la détection d'écart, profondeur, profil, forme, ondulation et/ou rugosité ou du trajet optique dans ou sur des objets techniques ou biologiques, et/ou pour la tomographie en cohérence optique (OCT) avec formation d'un interférogramme à lumière blanche spatial, comprenant :

mise à disposition d'un agencement d'interférométrie robuste à deux faisceaux selon une des revendications 1 à 10,
dans lequel une seconde détection optique du réflecteur terminal (111, 112, 113, 114) est réalisée au moyen d'un second interféromètre séparé avec une source de lumière laser pour pouvoir mesurer un coulissement dans la direction z de l'interféromètre (61) appartenant au réflecteur terminal (114) qui est connecté de manière rigide au réflecteur terminal (111, 112, 113, 114).

Figur 1

Figur 2a

Figur 2

**Figur 3**

Detail 4

Figur 4

28

Figur 5

Figur 6

Detail 7

Zoom B

Figur 7

Detail 8

Figur 8

**Figur 9**

Figur 10

Figur 11

Detail 10

EP 3 555 557 B1

34

Detail 12

Figur 12

Detail 13.1

delta_q

114

P''

Lbf

AHS

EHS

Lbf

Detail 13.2  Detail 13.3

Lbk

210

117

FO

20

FR

37

241

77

EB_1

B_117

B_114

beta_B

B_110

61

81

72

P''_entf

51

114

R

O

P''

P'

110

z

**Figur 13**

36

108

T_S

2A

x_S

**Detail 14**

rWLI
rWLI
rWLI
rWLI
rWLI
rWLI

Detektor-Koordinate

**Figur 14**

EP 3 555 557 B1

**IN DER BESCHREIBUNG AUFGEFÜHRTE DOKUMENTE**

**In der Beschreibung aufgeführte Patentdokumente**

- EP 2843360 A1
- EP 2526373 B1
- DE 102010056122 B3
- DE 102006007172 B4
- EP 2626373 B1